# EUROPEAN PATENT APPLICATION

(11) **EP 4 282 329 A1**
(43) Date of publication of application: **29.11.2023**
(21) Application number: 22174845.2
(22) Date of filing: 23.05.2022
(51) Int. Cl.: A61B 5/055, A61B 5/00, A61M 21/00

(54) **FMRI GUIDED COMA THERAPY OPTIMIZATION**

(71) Applicant: Koninklijke Philips N.V., 5656 AG Eindhoven (NL)
(72) Inventor: MENA BENITO, Maria Estrella, Eindhoven (NL); VAN EE, Raymond, Eindhoven (NL); LEUFKENS, Timmy Robertus Maria, 5656AG Eindhoven (NL)
(74) Representative: Philips Intellectual Property & Standards

(57) **Abstract**

The present invention relates to a device (122) for analyzing neural responses of a patient to different candidate treatment stimuli for treating a disorder of consciousness. An input (131) receives an fMRI timeseries of the patient and an output (132) provides a plurality of different stimuli, comprising sensory stimuli, at different points in time and/or in different combinations via a stimulus source (120) during the acquisition. These different stimuli correspond to different treatment conditions, and differ in the utilized stimulus source, the type or content of the stimulus and/or in stimulation parameter. The device comprises a second output (133) for outputting a value indicative of the neural response for each of the candidate treatments, and/or of a ranking or selection thereof. A processor (123) generates the value based on determined brain activity in response to each candidate treatment in (a) predetermined brain region(s).

## Description

### FIELD OF THE INVENTION

The present invention relates to the field of functional magnetic resonance imaging (fMRI), and particularly to the use of fMRI in image-guided therapies. The invention provides a device, system, method and computer-program product for image-guided therapy assessment (and/or therapy planning) using fMRI to assess the effect of different stimuli to treat a disorder of consciousness, e.g. to assist in determining a suitable therapy plan for coma patients.

### BACKGROUND OF THE INVENTION

Disorders of consciousness (DOC) refer to vegetative states (VS), minimally conscious states (MCS) and generally a wide spectrum of intermediate consciousness conditions characterized by a disruption and/or reduction of wakefulness and/or awareness. For example, coma, i.e. a deep state of prolonged loss of consciousness, is a severe disorder of consciousness. In this deep state of unconsciousness, the patient cannot be awakened, fails to respond normally to painful stimuli, light or sound, lacks a normal wake-sleep cycle and does not initiate voluntary actions. Coma patients exhibit a complete absence of wakefulness and are unable to consciously feel, speak or move. Coma can last from hours to even years, depending on the nature and severity of the underlying brain damage.

Clinically, a coma can be defined as the inability to consistently follow a one-step command. It can also be defined as a score of ≤ 8 on the Glasgow Coma Scale (GCS) over a period that lasts at least six hours. For a patient to maintain consciousness, the components of wakefulness and awareness must be maintained. Wakefulness describes the quantitative degree of consciousness, whereas awareness relates to the qualitative aspects of the functions mediated by the cortex, including cognitive abilities such as attention, sensory perception, explicit memory, language, the execution of tasks, temporal and spatial orientation and reality judgment.

From a neurological perspective, consciousness is maintained by the activation of the cerebral cortex, the gray matter that forms the outer layer of the brain, and by the reticular activating system (RAS), a structure located within the brainstem. During coma states there is a decrease in connectivity ("communication") between different cortical regions, or between cortical and subcortical regions, caused by a reduction of cerebral activity.

Since people in a coma are not able to express themselves, doctors must rely on a general physical examination and physical clues and information provided by families and friends. Routine bed examinations are done by means of standardized clinical behavioral scales. The commonly used clinical judgment scale is the Glasgow Coma Scale (GCS), which is composed of eye, verbal and motor tests. Although it is believed that clinical examination using the GCS coma score has a certain specificity for judging the prognosis of comatose patients, the GCS coma score is not very sensitive and can provide false positive results.

For the sake of simplicity, references to coma and DOC in the present disclosure may relate, generally, to patients diagnosed with a DOC, which can range in severity, e.g. from a complete and continual loss of consciousness over extended periods in time to states that allow intermittent episodes of (some) wakefulness or alertness. In other words, reference to coma may equally apply to other DOC conditions and/or vice versa.

Few effective and, even more importantly, reliable therapies are known for such conditions. Even though intensive care has seen substantial improvements in the past, e.g. such that it has become frequently possible for a patient to survive a very serious brain injury, advancements in therapeutical modalities to treat, and/or promote recovery from, a DOC caused by such injury have been lagging behind. Furthermore, prolonged intensive care and/or hospitalization needs can put a great burden on hospitals and society in general, e.g. the maintenance of life support for a coma patient can consume substantial social resources.

In many cases, a spontaneous awakening or spontaneous gradual improvements of consciousness and/or awareness offers the best, or even only, hope for recovery. Even though long-term coma is relatively rare, i.e. at least a partial recovery may typically occur within the first few months, such condition (and, by extension, also any less severe disorder of consciousness) can put an enormous strain on patients and, particularly, their family and close ones. The uncertainty associated with these disorders and the recovery process can be a source of substantial frustration and despair.

In the past, a treatment primarily consisted of maintaining the patient's physical condition and preventing complications, rather than focusing on the rehabilitation of the patient. If a patient does not show signs of spontaneous recovery in a relatively short time, currently known therapeutical options rarely can provide substantial prospects for restoration of consciousness, yet a hope for spontaneous recovery, even if uncommon, still remains, e.g. even after years of a stable lack of consciousness. Not only is recovery laced with uncertainty and may frequently only be very limited in scope, if achieved at all, regression of patients in responsiveness after a temporary recovery is a possibility as well, regardless of it being a spontaneous process or aided by therapy, such that a debilitating uncertainty in the family's shoulders can remain even after signs of considerable progress.

It can thus be easily understood that a need exists for effective and reliable early rehabilitation therapies for coma patients, e.g. in view of an advantageous reduction of the patient's time in a coma and improvement of the quality of life of the patient and his/her family.

The therapeutical options that are known and/or available, e.g. existing at various stages of maturity and/or availability in clinical practice, can be broadly classified as: pharmacologic therapies (in a broad sense, e.g. medicines promoting wakefulness, but also e.g. hyperbaric oxygen therapy), sensory stimulation, mechanical stimulation (e.g. particularly by focused ultrasound), electromagnetic stimulation and regenerative approaches (e.g. using stem cell therapy, neuro- and/or gliogenesis and/or axonal regrowth). Examples of electromagnetic stimulation include deep brain stimulation (DBS), intracranial electrical stimulation (IES), transcranial magnetic stimulation (TMS) and peripheral nerve stimulation (PNS). Combinations of different types of therapy can also be considered, e.g. pharmacologic stimulant therapies may be used to support another therapeutical modality, e.g. in an attempt to enhance the effect thereof. Rehabilitation therapies may also use sensory stimulation, e.g. by taking the patient through a regimen of controlled auditory (sound and/or more specific content, such as voice samples), visual (light and/or using image/video content) and/or other physical stimulation.

In the mechanical and electromagnetic stimulation modalities, which, generally speaking, typically involve a stimulation by physical waves or signals applied to the brain, invasive as well as non-invasive brain stimulation techniques can be used, e.g. to upregulate the pallido-thalamo-cortical circuitry of the brain via direct or indirect stimulation of the thalamic region. Invasive procedures may involve deep brain stimulation (DBS). For example, an electrode may be implanted to stimulate the thalamus, which can result in a patient regaining the ability of intelligible speech, or, more often, in limited improvements in motion control or in brain activity indicative or suggestive of (some level of) consciousness and/or awareness.

However, non-invasive stimulation may generally be preferable, particularly in view of the hit-or-miss odds of improvement and the risks involved in performing an invasive procedure (which may, moreover, be often complicated by a prior severe brain injury). For instance, transcranial direct current stimulation (tDCS) may be effective in sub-acute patients.

Low intensity focused ultrasound (LIFU) can also be used to modulate brain activity. By ultrasound stimulation of the thalamus (i.e. thalamic LIFU), some improvements in the level of consciousness have been shown to be achievable, at least for some patients suffering from acute DOC, e.g. the ability of understanding questions or instructions and responding thereto by deliberate motion, e.g. nodding or shaking the head, or reaching for items in the patient's environment, while the stimulation by ultrasound is relatively safe, particularly in comparison with direct electrical stimulation via implanted electrodes. One of the disadvantages is, for example, that it is difficult to deliver ultrasound stimulation frequently and/or over prolonged periods if time, due to the equipment requirements, if it is determined that continual stimulation is necessary to maintain an improvement in consciousness.

Currently the modality and parameters of such therapy, e.g. the selection and variety of stimuli and the area(s) to be stimulated, are mainly determined by trial-and-error and using behavioral responses without more objective assessment measures, e.g. real-time brain scanning at the bedside, as feedback to the clinical staff. Unfortunately, often a coma patient is not able to provide a behavioral response, even if the stimulation is being perceived and/or has some limited beneficial effect in view of (e.g. incrementally) improving the patient's condition. The effect of a therapy and the rehabilitation progress of coma patients is typically evaluated based on the GCS coma scale and the assessment of the attending therapist and medical personnel.

Over the past decades, increasing scientific attention has been focused on the study of coma using techniques such as electroencephalography (EEG), functional magnetic resonance imaging (fMRI), magnetoencephalography (MEG) and/or positron emission tomography (PET). Evaluation of coma by means of these techniques can provide a more objective measure of the consciousness of the patient. Such diagnostic imaging modalities and other relevant techniques (e.g. EEG) might be useful as guidance and/or assessment tool for a therapy. It has also been reported that the combined simultaneous acquisition of fMRI and PET may provide useful information for improving the characterization of patients with a disorder of consciousness.

Furthermore, the use of at least some form of imaging technology may be common in most invasive procedures, such as live/dynamic imaging to guide a surgeon to the correct target area in the brain during the implantation of electrodes, even though this is generally used more in the sense of an anatomical navigation tool than as a means for quantifying the effectiveness of the therapy or to obtain a measure that can be directly related to the effect of the therapy. Also, in non-invasive therapies, magnetic resonance imaging (MRI) may, for example, be used to aim the delivery of ultrasound waves to the targeted brain area, e.g. in MRI-guided thalamic LIFU. In such procedure, MRI guidance can be used to position a suitable ultrasound transducer in order to focus the emitted ultrasound waves, e.g. at a distance of about 5.5 cm from the surface, on (e.g.) the left central thalamus. MRI enables non-invasive imaging of internal body structures, with advantageously a good sensitivity and contrast in soft body tissues (e.g. the brain). A strong magnetic field is used to align the nuclear spins of atoms in the body, after which return signals emitted due to the relaxation of these spins can be studied in a location dependent manner, e.g. using suitable magnetic field gradients and purposefully designed radiofrequency excitation pulses. Many techniques exist in the art to prepare and/or process the observed signals, such as to create images that are sensitive to various material properties in the body.

Even though LIFU has potential as a safe and broadly applicable intervention for patients with chronic DOC, e.g. after months or even years of stable loss of consciousness, the delivery of an ultrasound therapy inside an MRI environment has several disadvantages. For example, the compatibility of the HIFU system with the MRI scanner can pose technical difficulties in view of the sensitive radiofrequency (RF) equipment and strong magnetic field (and field gradients) used by the MR system, which makes potential interference between the HIFU and MRI systems an important concern for safety of the equipment as well as of the patient. Furthermore, therapy inside an MRI system is difficult to maintain when the treatments are required over a prolonged time and/or frequently, in view of the costs involved, low availability of suitable systems and planning constraints on the MRI systems that are typically in high demand for various other examinations and procedures as well.

Since the options for analyzing the level of awareness and consciousness in DOC patients are quite limited, for obvious reasons, this complicates research in, and implementation of, therapies, e.g. it can be difficult to assess and quantify the response of a patient to a therapy. However, functional magnetic resonance imaging (fMRI) techniques offer a promising tool to detect and/or quantify brain activity, and can thus be useful for studying DOC patients. In fMRI, a detectable MRI image contrast is created that is indicative of blood oxygenation, e.g. using the blood oxygen level dependent (BOLD) effect. Thus, a hemodynamic response in active areas in the brain can be detected, such that it becomes possible to determine which areas in the brain are active (relative to a reference state). For example, specific sensory, cognitive, emotional, behavioral, motivational, memory and motor functions of the brain can be visualized and studied in detail using fMRI.

The use of sensory stimulation for coma and vegetative states has gained popularity during the last decades because of the flexibility and the variety of physical stimulation that can be delivered. Exposure to sensory stimuli, e.g. in a coma arousal technique, may allow the condition of a patient to improve, e.g. gradually over a prolonged therapeutical trajectory. Auditory stimuli may be provided by allowing a family member, e.g. family members one at a time, to (verbally) interact with the subject. For visual stimulation, a subject may be surrounded by light (of various qualities, e.g. color, intensity, ..., and dynamics, e.g. static, strobing, ...), contentful visual information, e.g. brightly colored pictures, family photos, ..., and/or familiar objects placed in the visual field.

Touch stimuli can range from light touches to pain signals. Even though the tactile sense would appear relatively simple compared to other senses, stimuli can still vary over many parameters, e.g. in locality, textural aspects, dynamics, diversity, intensity, etc. For example, the tactile sense is composed of at least six different receptor pathways that detect (without straightforward decomposition at the receptor level) aspects of fine touch, vibrations and other dynamics, pain, skin stretching, temperature (heat and cold), surficial pressure phenomena and deep pressure (e.g. important for proprioception). A variety of textures can be used for tactile stimulation, such as clothing, blankets, lotions etc. Massage techniques (e.g. squeezing) may also be applied. In addition to the tactile system as such (in its narrowest sense), the proprioceptive, vestibular and interoceptive systems may also be stimulated to arouse the patient. For example, movement and kinesthetic stimulation can also be used. For example, range of motion exercises and changes in position may stimulate the patient's brain and may even be required regardless of a therapeutical utility for maintaining the physical health of the body.

Olfactory stimuli (and/or, in a wider sense, chemo-sensory stimuli) offers another potentially useful route, such as by exposure to the smell of perfumes, coffee powder, shampoo, foods and/or other products with a remarkable (or even pungent) odor. Even though often underestimated, the brain has evolved around a strong and short (direct) connection to the olfactory sense, such that familiar odors, e.g. associated by the patient with memories and/or emotions, might have a strong arousing effect on the brain. Furthermore, the olfactory system has an amazing complexity and is very difficult to decompose in a system of base sensory components. This can create substantial challenges in finding and manipulating odors that can have a therapeutic effect, and studying the effects thereof in a systematic, quantitative and/or objective manner.

Moreover, multimodal sensory stimulation and/or variety of stimulation (e.g. over time) may play an important role in achieving an effective arousal and enhancing clinical outcomes for people in coma. However, it will be understood that, given the wide range of potential sensory stimulation types and potential stimulation content, a combinatorial explosion of options when considering (e.g. multimodal) combinations, and possibly a benefit of novelty (e.g. frequent changes in the provided stimuli over time), it may be difficult to determine an effective strategy, and even more challenging to devise a strategy optimized as function of the clinical outcome. Currently, sensory stimuli are typically administered through multiple sensory channels and at a moderate to high intensity. However, even though sensory stimulation can often promote positive outcomes, patients that are exposed to an undifferentiated bombardment of sensory information may also lose the ability to process such information by habituation and/or due to sensory overload of the limited capacities of the injured brain.

### SUMMARY OF THE INVENTION

It is an object of embodiments of the present invention to provide in good, effective and efficient means and methods to determine, e.g. quantify, and/or analyze the response of a patient to different candidate stimulation treatments for disorders of consciousness (DOC), and/or to optimize treatment parameters and/or select a suitable therapy or therapies, i.e. provide a suggestion or selection of promising candidate treatments to the attending clinical staff to support the planning and/or finetuning of a therapeutical trajectory.

Embodiments of the invention can be used for supporting the rehabilitation of DOC (e.g. coma) patients by optimizing a stimulation treatment, e.g. optimizing the selection, order, frequency, duration and/or other parameters of stimulation. Tailoring the treatment to the individual needs of the patient and the objectively detected evoked response to the treatment may result in a more efficient and/or successful rehabilitation process.

It is an advantage of embodiments of the present invention that a neural response to therapeutical stimuli can be determined, even in the absence of directly observable (e.g. by direct human observation) results, such as a cognitive response, an emotional response, an attention to the delivered stimuli and/or any other change in brain activity indicative of consciousness and/or alertness.

It is an advantage of embodiments of the present invention that a patient suffering from a DOC, e.g. in a vegetative state, minimally conscious state or other state of impaired wakefulness and/or awareness, can be treated so as to optimize, or at least attempt to improve, treatment outcomes, e.g. to improve the reliability of a therapy.

It is an advantage of embodiments of the present invention that a burden to society and a patient's family can be reduced by finding and/or optimizing a rehabilitation therapy for a coma/DOC patients, by potentially reducing a patients' time in a coma and/or improving the quality of life of the patient.

It is an advantage of embodiments of the present invention that a strain on intensive care or prolonged hospitalization resources can be reduced by improving the odds of (partial or complete) recovery in DOC patients.

It is an advantage of embodiments of the present invention that a personalized treatment strategy can be determined, e.g. optimized to aim for good results in treating a specific patient.

It is an advantage of embodiments of the present invention that an optimized treatment can reduce the demand for costly or not easily availability treatment modalities. For example, when a therapeutical modality is determined to be less, or likely not, effective, time and resources that could be substantially wasted by treating a patient by such means can be reduced or avoided.

It is an advantage of embodiments of the present invention that changes in a patient's state, and response to specific therapies, can be taken into account by repeatedly updating a treatment strategy to optimize outcomes. For example, a patient who at the onset of a treatment trajectory would not respond favorably to stimuli of a particular nature, might benefit from exposure to such stimuli when, at a later point in time, his/her condition has improved to the level of sufficient responsiveness. By tuning a treatment to a quantifiable response in the patient's brain, situations in which the wrong type of stimulation is provided at the wrong time, and therefor wastage of resources and non-optimal recovery, can be avoided or reduced. Likewise, the disadvantageous effects of habituation to a therapy can be reduced or avoided by finetuning and/or adjusting a therapy based on the objective information that can be obtained via embodiments of the present invention.

It is an advantage of embodiments of the present invention that incremental improvements in a patient's condition, even if not directly perceivable (by direct human observation), can be monitored and followed-up overtime. This may also reduce the burden on a patient's close ones, since uncertainty with respect to the patient's condition and prospects of future recovery may be reduced.

It is an advantage of embodiments of the present invention that a relapse of the brain's response to a treatment plan can be detected early on, such that the therapy can be adjusted to avoid, or reduce the risk of, regression of the patient.

It is an advantage of embodiments of the present invention that (e.g. recurrent) therapy sessions may be performed outside an magnetic resonance imaging (MRI) environment, while an initial (and/or follow-up) treatment tuning session can be performed in MRI. This enables a patient-specific therapy, while still allowing for an efficient use of resources.

For example, when a sensory stimulation is identified by functional MRI (fMRI) analysis that is effective in evoking a neural response, stimulation sessions with such easily accessible and cost-effective means can be scheduled outside the MRI environment. This can be followed up, e.g. after some time and/or after some number of sessions, by a new fMRI examination to review the patient's progress and/or to tune the therapy to account for possible changes in the patient's condition and/or responsiveness to therapies.

Patients given sensory stimulation therapy have been found to show earlier recovery of consciousness than those without such stimulation. The use of multi-therapy and a variety of multisensory stimulations have also been demonstrated to be more efficient than the use of a single type of sensory modality. Therefore, it is an advantage of embodiments that such sensory therapies can be optimized in an objective and patient-specific manner. In the present state of the art, the nature and selection of variety of stimuli may be commonly established by trial-and-error, using behavioral responses without objective real-time brain scanning. Embodiments of the present invention provide the advantage of providing a more objective and sensitive quantification and/or detection of therapy response and/or improvements of the patient's condition, even if not directly observable (e.g. by behavioral changes).

It is also an advantage that sensory stimulation means may be relatively cheap, simple to use and readily available, e.g. compared to direct/indirect brain stimulation (IES, DBS, TMS, HIFU, ...). The response to a type of treatment that relies on less easily available equipment, e.g. HIFU, TMS, ..., may also (additionally) be tested in accordance with embodiments of the present invention. Even though this may require a more complex configuration, specifically designed/modified hardware, e.g. a dedicated (e.g. MRI-safe) ultrasound transducer and/or TMS coil, and/or extensive session preparations to be able to combine such treatment modality (or modalities) with MR imaging, it remains an advantage that the therapy delivery can subsequently take place outside the MRI system, e.g. such that in (e.g. the majority of) therapy delivery sessions a standard transducer or TMS coil can be used, the complexity of the session preparation can be reduced and/or the therapy session can be carried out by personnel that does not need specific training in MRI safety procedures. Particularly, if the response to a sensory stimulation program is found to be more or substantially equally effective, unnecessary costs, wasted resources and effort can advantageously be avoided, or a combination therapy can be identified that may obtain even better results.

It is also to be noted that if a therapy is found to be effective, e.g. to maintain or stabilize an improvement in consciousness, (e.g. only) when it is maintained over prolonged periods of time (per session and/or cumulative) and/or when applied frequently, a strain on MRI system resources can still be kept relatively low by occasionally (e.g. infrequently) scheduling an fMRI follow-up to monitor the patient's continual response to the therapy and/or to test the effect of alternative, possibly less demanding, treatment modalities.

For some treatment modalities, such as HIFU and/or TMS, embodiments of the present invention may be conveniently combined with (an)other MRI protocol(s) to correctly position and/or focus a transducer, coil or similar treatment delivery device in a same MRI session. In other words, some treatments may already require an MR imaging examination to determine a suitable position on the scalp, such that an fMRI test in accordance with embodiments can be applied without a large additional cost in resources.

Even though embodiments of the present invention may be used to determine a suitable program for therapy outside the MRI environment, e.g. in a number of repeat sessions before a return for re-evaluation and/or finetuning the program, it is also an advantage that in accordance with embodiments of the present invention a stimulation therapy and the assessment of the effect thereof on the patient can be performed in parallel (substantially simultaneously), e.g. at least during the assessment session (which may be combined with follow-up sessions outside the MRI environment, or not). Current approaches may typically rely on (a) therapy session(s) followed by a separate examination and/or evaluation, possibly with a substantial time window in between. By following up on the progress of the patient in response to the stimulation therapy in real-time, or at least at a high review frequency, i.e. as facilitated by embodiments of the present invention, the attending clinicians can get insights into the effectiveness of the treatment and can be aided in optimizing the strategy. Furthermore, by substantially providing the stimulation under test and the fMRI detection of brain response in parallel and/or simultaneously (at least when ignoring negligible time delays in view of design paradigm scheduling, technical limitations and latency of the detectable brain response), a high sensitivity to transient and/or subtle effects in the brain can be achieved. Such transient effect may be still relevant if it can be used to achieve incremental improvements in the patient's condition, but would, in a more conventional, sequential approach of therapy and assessment remain undetected.

A device, system, method and computer-program product in accordance with embodiments of the present invention achieves the above objective.

In a first aspect, the present invention relates to a method for analyzing neural responses of a patient to a plurality of different candidate treatment stimuli for treating a disorder of consciousness using functional magnetic resonance imaging. The method comprises acquiring a timeseries of functional magnetic resonance images of the brain of the patient using a magnetic resonance imaging system. The method comprises providing a plurality of different stimuli via one or more stimulus sources, at different points in time and/or in different combinations, to the patient during the acquisition of said timeseries to induce activity in the patient's brain. The plurality of different stimuli comprises sensory stimuli, and the different stimuli correspond to different candidate treatment conditions. The treatment stimuli differ in the stimulus source that is used, in the type of stimulus and/or in a parameter of the stimulus. It will be understood that each of the different stimuli may typically also be repeated, e.g. providing a plurality of trial stimulations of the (and each) same type of stimulus and/or corresponding to the same parameter selection so as to enable a detection of the effect of each candidate treatment condition based on a joint analysis of the induced brain activity by these trial stimulations.

The method further comprises determining an activity measure indicative of brain activity in response to each candidate treatment condition (e.g. determining an activity measure for each candidate treatment condition) in at least one predetermined region of interest in the patient's brain based on said timeseries. The method comprises outputting at least one value, based on these activity measures, that is indicative of the neural response for each of the candidate treatments, of a ranking of the candidate treatments (e.g. ordered as function of the activity measure), and/or of a selection of said candidate treatments (e.g. a list of a predetermined number of the candidate treatments for which the determined activity measures are the highest).

In a method in accordance with embodiments of the present invention, the plurality of different stimuli may comprise visual, auditory, tactile, haptic, olfactory and/or other sensory modality signals that are suitable for being directly perceived by the brain via the patient's sensory organs. In a method in accordance with embodiments of the present invention, the visual signal may comprise a light signal, an image, a photograph and/or a video component of a movie clip. In a method in accordance with embodiments of the present invention, the auditory signal may comprise an audio tone, a sound, an ambient audio recording, a voice recording and/or an audio component of a movie clip. In a method in accordance with embodiments of the present invention, the olfactory signal may comprise a signal delivered by a time-controlled exposure of the patient to an odorous compound via mouth or nose.

In a method in accordance with embodiments of the present invention, the plurality of different stimuli may be distinguished from each other based on their sensory modality and/the or specific combination thereof, on a content type of content delivered via the stimulus, on a patient-specific level of memory association for the content based on memories of and/or on familiarity to the patient, on a grading of the content as function of arousal, valence and/or another psychological grading parameter(s), and/or on one or more technical parameters of the stimulus delivery.

In a method in accordance with embodiments of the present invention, providing the plurality of different stimuli may comprise, e.g. in addition to providing sensory stimuli, providing stimuli to directly or indirectly modulate activity of the brain by electric, magnetic and/or mechanical stimulation of brain tissue.

In a method in accordance with embodiments of the present invention, each of said plurality of different stimuli may be provided at a plurality of different times during said acquisition in accordance with a functional magnetic resonance imaging block paradigm design and/or a functional magnetic resonance imaging event-related paradigm design.

In a method in accordance with embodiments of the present invention, determining said activity measure may comprise fitting a general linear model, generalized linear model and/or statistical model to said timeseries.

In a method in accordance with embodiments of the present invention, determining said activity measure may comprise evaluating a trained machine learning model with said timeseries and/or information derived therefrom as input and said activity measures and/or said output value as output. A method in accordance with embodiments of the present invention may comprise obtaining, during and/or after the fMRI acquisition, assessment data from the patient, in which the assessment data comprises face reaction data, eye movement data, pupil dilation data, muscle reaction data physiological data and/or clinical behavioral scoring data. The method may comprise taking said assessment data into account in determining the at least one value for output.

A method in accordance with embodiments of the present invention may comprise obtaining further imaging and/or physiological data (e.g. repeatedly, e.g. so as to enable the use of this data in detecting neuronal effects of the, e.g. for each of the, different candidate treatments) during said acquisition using a non-magnetic-resonance diagnostic imaging modality, Positron Emission Tomography, electroencephalography and/or magnetoencephalography, and taking said further imaging and/or physiological data into account in determining said activity measure.

In a method in accordance with embodiments of the present invention, the at least one predetermined brain region may be associated (e.g. based on scientific knowledge available in the art) with consciousness, awareness, memory, emotion and/or any other higher brain function.

In a method in accordance with embodiments of the present invention, the at least one predetermined brain region may comprise one or more of: the reticular activating system, the anterior cingulate cortex, the thalamus, the caudate nucleus, the hippocampus, the parahippocampus, the fusiform gyrus, the posterior cingulate gyrus, the precuneus, one or more regions of the frontal cortex, the parietal cortex and/or the temporal cortex, the posteromedial cortex, the dorsolateral prefrontal cortex, and/or the medial temporal cortex, and/or parts and/or combinations thereof. Each of these illustrative regions may be considered unilaterally (specifically the left or right structure) and/or bilaterally (as a single region of interest or as separate regions of interest), where applicable (e.g. for symmetric structures).

In a method in accordance with embodiments of the present invention, determining the activity measure may comprise performing a neuronal network activation analysis to determine a measure indicative of a relation between activity in different predetermined brain regions. For example, the neuronal network activation analysis may be performed for the medial frontoparietal network (e.g. the default mode network), the frontoparietal network (e.g. the central executive network), the dorsal attention network and/or the ventral attention network, and/or parts and/or combinations thereof.

A method in accordance with embodiments of the present invention may comprise obtaining a reference brain map of the patient to locate said at least one predetermined brain region using anatomical mapping based on an anatomical reference magnetic resonance image obtained from the patient.

A method in accordance with embodiments of the present invention may comprise obtaining a reference brain map of the patient to locate said at least one predetermined brain region using tractography based on diffusion-weighted images obtained from the patient.

In a method in accordance with embodiments of the present invention the output value may comprise a prognosis indicator indicative of a prognosis of the patient as determined by taking the output value and/or activity measures determined in a previous session of performing the method for the same patient into account.

In a method in accordance with embodiments of the present invention the steps of determining the activity measure may be performed during the acquisition so as to progressively recalculate and/or update the activity measures.

A method in accordance with embodiments of the present invention may comprise optimizing the plurality of different stimuli and/or a scheduling thereof based on the progressively updated and/or recalculated activity measures, so as to adapt the sequence of stimuli yet to be provided.

In a second aspect, the present invention relates to a computer-program product adapted to perform a method in accordance with embodiments of the first aspect of the present invention.

In a third aspect, the present invention relates to a device, e.g. a computer system, for analyzing neural responses of a patient to a plurality of different candidate treatment stimuli for treating a disorder of consciousness. The device comprises an input for receiving a timeseries of functional magnetic resonance images of the brain of the patient acquired using a magnetic resonance imaging system. The device comprises a first output for providing a plurality of different stimuli at different points in time and/or in different combinations via one or more stimulus sources, e.g. external to the device (e.g. connected via a suitable interface) and/or comprised in the device, to the patient during the acquisition of the timeseries to induce activity in the patient's brain. The plurality of different stimuli comprises sensory stimuli. The different stimuli correspond to different candidate treatment conditions. The different treatment stimuli differ in the stimulus source that is used, in a type of the stimulus and/or of its content and/or in a parameter of the stimulus.

The device comprises a processor for determining an activity measure indicative of brain activity in response to each candidate treatment condition in at least one predetermined region of interest in the patient's brain based on the received timeseries.

The device further comprises a second output for outputting at least one value that is indicative of the neural response for each of the candidate treatments, of a ranking of said candidate treatments, and/or of a selection of said candidate treatments. The processor is adapted for generating and outputting said at least one value, via the second output, based on the determined activity measures for the candidate treatment conditions.

A device in accordance with embodiments of the present invention may comprise said one or more stimulus sources.

In a device in accordance with embodiments of the present invention, said one or more stimulus sources may comprise a visual, auditory, tactile, haptic, olfactory and/or another sensory modality stimulator.

In a device in accordance with embodiments of the present invention, the stimulus sources may further comprise a direct or indirect brain stimulator to modulate activity of the brain by electric, magnetic and/or mechanical stimulation of brain tissue.

In a device in accordance with embodiments of the present invention, the plurality of different stimuli may be distinguished from one another (i.e. may differ in) based on their sensory modality and/or specific combinations thereof, on a content type of content delivered via the stimulus, on a patient-specific level of memory association for the content based on memories of or familiarity to the patient, on a grading of the content as function of arousal, valence and/or another psychological grading parameter, and/or on one or more technical parameters of the stimulus delivery.

In a device in accordance with embodiments of the present invention, the processor may be adapted to determine the activity measures by fitting a general linear model, generalized linear model and/or statistical model to the received timeseries.

In a device in accordance with embodiments of the present invention, the processor may be adapted to determine the activity measures by evaluating a trained machine learning model with the received timeseries and/or information derived therefrom as input and the activity measures and/or the output value as output.

In a device in accordance with embodiments of the present invention, the processor may be adapted to determine the activity measure in the at least one predetermined brain region associated with consciousness, awareness, memory, emotion and/or a higher brain function, and/or combinations thereof.

In a device in accordance with embodiments of the present invention, the processor may be adapted to determine the activity measure in the at least one predetermined brain region comprising one or more of: the reticular activating system, the anterior cingulate cortex, the thalamus, the caudate nucleus, the hippocampus, the parahippocampus, the fusiform gyrus, the posterior cingulate gyrus, the precuneus, one or more regions of the frontal cortex, of the parietal cortex and/or of the temporal cortex, the posteromedial cortex, the dorsolateral prefrontal cortex, and/or the medial temporal cortex.

In a device in accordance with embodiments of the present invention, the processor may be adapted to perform a neuronal network activation analysis to determine the activity measure.

In a device in accordance with embodiments of the present invention, the processor may be adapted to locate said at least one predetermined brain region based on a reference brain map of the patient determined from an anatomical reference magnetic resonance image of the patient (e.g. received via the first input in addition to the fMRI timeseries) and/or a tractography analysis of diffusion-weighted images of the patient (e.g. received via the first input in addition to the fMRI timeseries).

In a device in accordance with embodiments of the present invention, the processor may be adapted to generate a schedule for providing the plurality of different stimuli via the first output.

A device in accordance with embodiments of the present invention may comprise a further input for receiving, during and/or after receiving the timeseries, i.e. during and/or after the acquisition, assessment data and/or physiological data of the patient. The assessment and/or physiological data may comprise clinical behavioral scoring data, face reaction data, muscle reaction data, eye movement data and/or pupil dilation data, and/or physiological data of the patient. The further input may also be adapted, additionally or alternatively, to receive further imaging data. The processor may be adapted to take the assessment, physiological and/or further imaging data into account in determining said activity measure and/or said output value.

In a device in accordance with embodiments of the present invention, the processor may be adapted to determine said activity measure during the timeseries acquisition, so as to progressively recalculate and/or update the activity measures.

In a device in accordance with embodiments of the present invention, the processor may be adapted to optimize the plurality of different stimuli, and/or a scheduling thereof, based on the progressively updated and/or recalculated activity measures so as to adapt the sequence of stimuli yet to be provided.

In a fourth aspect, the present invention relates to a magnetic resonance imaging system, e.g. adapted to perform a method in accordance with embodiments of the first aspect of the present invention, wherein said system comprises a device in accordance with embodiments of the third aspect of the present invention.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a system in accordance with embodiments of the present invention.
Fig. 2 shows a method in accordance with embodiments of the present invention.
Fig. 3 shows a device in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

References to a 'stimulus' refer to external signals that are applied to a patient to evoke a specific response in the patient's brain.

Such response is typically localized in time, e.g. occurs at the moment when the stimulus in provided or shortly thereafter. Nevertheless, the stimulus may also have effects on a longer time scale, e.g. an improvement of the patient's condition (e.g. awareness, lucidity, ...) over a longer time, e.g. minutes, hours, days or even longer after the application of the stimulus. While the latter, long-lived response may be the desirable outcome of a successful stimulation therapy, the former (direct) response, i.e. the substantially immediate response in the brain, e.g. on a scale of a second up to minutes after the stimulus (or a sequence of stimuli), is used in embodiments of the present invention to detect and quantify a potential use, suitability and/or efficacy of the stimulus (or of a combination, sequence or other composition of such stimuli) as a basis for a therapy.

Thus, the (direct) stimulus response has a specific temporal relationship to the time of providing the stimulus. The response of interest is, in a sense, also localized in space, e.g. occurs in a specific brain region or regions, along predetermined neural pathways, or can similarly be identified in the spatiotemporal fMRI data. Without necessarily excluding stimuli that involves an action of the patient, e.g. within the limited capabilities of the impaired DOC patient (in less severe cases), the stimulus is typically limited to the patient's passive observation of, or being subjected to, the stimulus.

Specifically, the stimulus may be a sensory stimulus, e.g. which may evoke (at least if the candidate therapy relying on this type or configuration of the stimulus is effective) a response in brain regions linked to consciousness and/or awareness in the patient via one or more sensory organs and neural pathways. Stimuli may also (i.e. additionally to sensory stimuli) be delivered directly or indirectly to the patient's brain, e.g. via an electromagnetic and/or mechanical signal, such as by transcranial magnetic stimulation, deep brain stimulation and/or ultrasound stimulation of brain tissue.

The neural response of interest may involve an emotional response, a mood shift, a sentiment, a memory, conscious thought or the like, whereas responses that are typically not (or to a lesser extent) of interest may involve autonomous, subconscious and/or involuntary activity in the brain, e.g. activity of brain areas limited to only the direct mechanics of visual, auditory or other sensory reception and/or are not related to the intended effect of the stimulation as such.

In a first aspect, the present invention relates to a method for analyzing the effects of (the neural response to) a plurality of different stimuli for treating a disorder of consciousness (DOC), e.g. to assist a clinician in determining a suitable therapy plan guided by information obtained by functional magnetic resonance imaging (fMRI). The method may particularly be a computer-implemented method, e.g. performed by a computer program product when executed on a suitable computing platform. For example, the method may be implemented by software and/or hardware, e.g. completely or partially implemented in (e.g. dedicated) computing hardware, e.g. digital processing hardware.

Particularly, the method may be adapted to monitor and/or determine the effect of a stimulation therapy on a patient, to evaluate evoked signs of consciousness, to quantify the effect of the stimuli, e.g. on a rating scale to assess the effect and effectiveness of a sensory stimulation therapy, and/or provide, optimize and/or personalize a stimulation therapy program (or, at least, create a computer-aided suggestion the attending physician).

The response of a patient to different candidate treatments (e.g. in which each candidate treatment consists of the same or similar type of stimuli, but differs in a parameter or other quality from other candidate treatments) for disorders of consciousness (DOC), e.g. as a result of a severe brain injury, can be analyzed and/or a suitable therapy can be selected (and/or adjusted), aided by the method in accordance with embodiments. For example a treatment can be selected from a plurality of options explored by the method and/or treatment parameters can be optimized based on the observed brain response.

The method may be performed by interacting with a magnetic resonance imaging system.

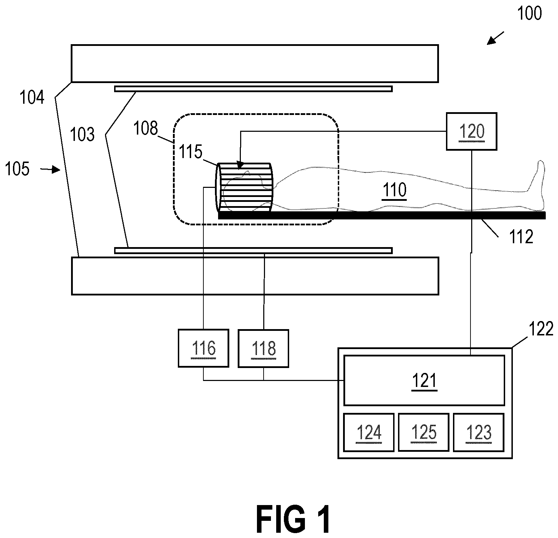

Fig 1 shows an example of such magnetic resonance imaging system 100, e.g. an MRI system that is generally suited to implement steps of a method in accordance with embodiments of the present invention. In a further aspect, the present invention relates to such MRI system 100 adapted to perform a method in accordance with embodiments of the first aspect of the present invention.

The MRI system 100 comprises a magnet 104, e.g. a superconducting cylindrical magnet through which a bore 105 is defined. Variations of and/or alternatives for this type of magnet are also possible, e.g. the magnet may be a split cylindrical magnet or an open magnet (e.g. in which magnet sections are arranged around, e.g. above and below, an open space for receiving the subject to be imaged). The magnet 104 may comprise a plurality of superconducting coils, e.g. in a cryostat. Inside the bore 105, an imaging region 108 is defined in which a subject can be imaged. In this imaging region 108, the magnetic field of the magnet 104 is typically strong and uniform, i.e. sufficiently strong and uniform to perform MR imaging. For an open magnet or other alternative magnet assembly, there might not be a bore cavity defined, but the imaging region 108 will still be clearly identifiable, e.g. as characterized by a suitable magnetic field strength and homogeneity. The magnetic resonance data acquired by the system, in use thereof, is typically representative of the content of the space defined by the imaging region 108, or a part thereof, e.g. a specific region of interest defined inside this imaging region.

A patient 110 can be, in use, placed inside the imaging region 108. For this, the system may comprise a subject support 112, e.g. an actuated patient couch. By controlling the subject support, a body part or region of interest in the subject on the support can be moved to a suitable position inside the imaging region 108.

The system may further comprise a plurality of magnetic field gradient coils 103, adapted to spatially encode magnetic spins within the imaging region 108 (e.g. by superimposing local magnetic field variations onto the strong magnetic field of the magnet). For example, the magnetic field gradient coils may comprise at least three coils (or sets of coils) to allow spatial encoding in three orthogonal spatial directions, i.e. in any direction in space. The magnetic field gradient coils 103 may be controlled as a function of time, in addition to control such as to select the spatial encoding direction, e.g. to create gradients that are pulsed, ramped or otherwise varying as function of time. For example, a power supply of the magnetic field gradient coils may be controlled by a controller, processor or computer to achieve a magnetic field (gradient) in accordance with a desired spatial and/or temporal function. The system may typically comprise a controller 118 to control the magnetic field gradient coils 103.

The system may typically comprise a radiofrequency (RF) coil 115 (an RF antenna device) which may act as a radiofrequency receiver and transmitter. For example, the RF coil may transmit a signal that disrupts the alignment of (nuclear) spins with the magnetic field (of atoms and molecules in the imaging region 108), and the RF coil may receive a return signal that is generated by a precession and relaxation of the disrupted nuclear spins back to the magnetic field alignment. The RF coil may be a construct comprising a plurality of separate coil elements, and/or support equipment. The RF coil may be integrated in the bore system, and/or may be a separate component to be placed close to or in contact with the imaged patient. For example, the RF coil(s) may be integrated in a head coil assembly for imaging the brain of the subject 110.

The RF coil 115 comprises, or is connected to, an RF transceiver 116. Obviously, the system may also comprise separate RF transmission and receival antennas and corresponding (separate) receiver and transmitter systems, or any combination of a plurality of antenna coils and transceiver and/or receiver and/or transmitter systems. For example, a plurality of antenna coils may be connected to separate receive and/or transmit channels, which may be controlled separately, in unison or in any combination that is considered suitable for the intended purpose. A number of parallel channels may, for example, be useful for parallel imaging techniques, such as SENSE or ASSET (sensitivity encoding; array coil spatial sensitivity encoding).

The MRI system 100 may also comprise a stimulus source 120 to provide a stimulus to the patient during an MRI imaging operation. The stimulus source(s) 120 specifically comprises one or more sensory stimulus sources, to provide a signal (stimulus/stimuli) to the patient via any of (or combination of) the physical senses of the body, such as touch, smell, taste, hearing and sight. The sensory stimulus source may include a light(s) and/or display to present a visual stimulus to the patient while positioned in the imaging region (e.g. including possibly a projection screen and/or optical system to relay displayed information to within the bore volume), and/or a speaker or other sound source to present an auditory stimulus to the subject (including possibly a relay system, e.g. using guided air pressure transmission or contact conduction of sound into the bore volume, e.g. to a headphone). Other examples include means to provide a somatosensory stimulus to the patient, such as a haptic/tactile signal, a system to control a (perceivable) directed airflow onto the body of the patient, a device to deliver a pain signal to the body, a heating and/or cooling device to deliver a perceivable change in temperature (or heat flux) to the body (or a specific part thereof), and/or generally any means suitable to deliver a signal to the patient that is (ideally) perceivable by the patient's senses. Optionally, the stimulus source 120 may also comprise one or more devices for directly (but not necessarily invasively) stimulating neural tissue in the patient's brain, e.g. a TMS coil (and/or system), a focused ultrasound transducer (and/or system) and/or other such devices for stimulating the brain (preferably in a well-controlled specific region), e.g. such as to enable the comparison of its effect to the effect of sensory stimulation types and/or explore combination therapies using direct brain stimulation and sensory stimulation.

The stimulus source(s) 120 is (are) used, in performing a method in accordance with embodiments, to trigger activity in one or more brain regions (or brain circuits) of interest in the subject, e.g. that are implicated in consciousness and/or awareness. Even though, generally, the stimulus may be delivered to the patient in a passive state, e.g. would not require an active response of the patient in view of his/her condition (however, not necessarily excluding more active options, e.g. where the DOC condition enables some limited, transient episodes of awareness, some limited control of the body, or the like), a stimulus, such as an auditory or visual instruction to the patient to take a specific mental action in response to the signal are also possible. For example, the patient may be requested to indicate his/her understanding of the instruction by attempting to focus thought, by attempting to move a part of the body (even if his/her condition prohibits actual movement), to remember specific events (or memories of a predetermined type), and/or to undertake any other mental activity that would indicate a conscious response or condition in the patient.

The timing of the stimuli may be controlled with respect to the timing of the collected MRI data, such that acquired images can be analyzed by taking the stimuli presented at the time of image collection into account (or prior to the time of image collection by an at least approximatively known time delay).

The RF transceiver 116, the gradient coil controller 118 and the stimulus source(s) 120 may be connected to a hardware interface 121 of a computer system 122. The hardware interface may, for example, be a digital network interface or data bus interface. The hardware interface may be adapted for exchanging data and/or commands between the computer system and other components of the system. The hardware interface may also be adapted for exchanging data and/or commands between different components of the computer system. The hardware interface is not necessarily a homogenous interface infrastructure, e.g. may comprise or consist of different types of data exchange media, computer networks, communication buses, shared memory access structures and the like, and/or may be distributed over a plurality of processing devices.

The MRI system 100 is, particularly, adapted for acquiring fMRI images. In other words, a timeseries of images can be acquired, in which each image in the sequence is reconstructed, e.g. by the computer system 122, based on signals detected by the RF transceiver and the antenna coil(s) connected thereto. For fMRI imaging, the RF coil(s) may be, specifically (or, at least, typically), integrated in a head coil assembly to achieve a good signal quality for imaging the patient's brain.

Embodiments in which the computer system comprises a plurality of computers, interconnected by a suitable network infrastructure are not necessarily excluded. For example, processing performed by the computer system may be distributed over a computing cluster or cloud computing platform. Also, while reference is made to a conventional computer architecture hereinbelow, it will be clear to the skilled person that this is not necessarily the case in all embodiments. For example, the computer system may comprise, or even consist of, dedicated hardware for implementing the processing functions described hereinbelow, e.g. comprising an application specific integrated circuit and/or field-programmable gate array hardware. It will also be understood that, where reference is made to a single computer system, different functions of this computer system may be assigned to dedicated computer systems, e.g. which are able to exchange data. A distributed computer (and/or other processing and/or control) infrastructure, e.g. for controlling different aspects of the operations, may be adapted to use a central reference clock signal or may use similar synchronization means to enable (different parts of) the system to control and/or account for the relative timing of stimuli, gradient and/or RF signals and data acquisitions.

The computer system 122 may comprise at least one processor 123. The processor may be operably connected to the hardware interface, such as to allow the gradient coils, the sensory stimulus source and the RF transceiver to be controlled by the processor and/or to enable processing of data received by the RF transceiver by the processor.

The computer system 122 may comprise a user interface 124, e.g. for interacting with an operator operating the MRI system. The processor may likewise be connected to the user interface, e.g. such that a user can select actions to perform by the MRI system, parameters to apply, review data indicative of the operation of the system and/or images as acquired by the system (or images or data, e.g. output data, derived therefrom). The user interface may comprise a display, a keyboard, a pointer device, a touch interface, and/or other such components as are ubiquitous in human-computer interaction systems. The user interface may be adapted to render images, e.g. via the display.

The computer system 122 may comprise a memory 125. The memory may comprise or consist of different types of memory, all of which are (directly or indirectly) accessible to the processor. This may for example include a random access memory, a cache memory, non-volatile memory (such as flash memory), hard drives, solid state drives, and/or other storage devices. The memory may comprise a non-transitory computer-readable medium.

The memory may contain machine-executable instructions to enable the processor to perform various data processing tasks and/or to control other components of the MRI system. The machine-executable instructions may enable the processor to perform at least part of a method in accordance with embodiments of the present invention.

A method in accordance with embodiments of the present invention may be implemented by a source program, an executable program (e.g. a bytecode or intermediate language code, an object code and/or a machine code), a computer-interpretable script and/or any other entity comprising a set of instructions that can be directly or indirectly (e.g. by means of facilitating programs or codes, such as interpreters, just-in-time compilers, compilers, etc., and/or supporting code libraries, frameworks, an operating system, etc.) executed by a computer system, e.g. by the processor of the computer system 122. It is pointed out that embodiments are not limited to computer code implementations, e.g. the method may be performed (completely or in part) by dedicated hardware specifically designed for the intended method, e.g. in an ASIC, and/or by a hardware configuration that can be loaded in a programmable hardware construct, e.g. an FPGA.

In further aspects, the present invention relates to a device, e.g. the computer system 122 and/or such specifically adapted processing hardware, adapted to perform a method in accordance with embodiments of the first aspect of the present invention, and to a computer-program product adapted to perform a method in accordance with embodiments of the first aspect of the present invention when executed by a computer.

The memory 125 may also contain a representation of pulse sequence commands, which enable the processor to control the MRI system in a corresponding manner, e.g. by controlling the gradient coils and RF transceiver in accordance with these commands. The memory may also contain magnetic resonance imaging data acquired by controlling the MRI system with the pulse sequence commands.

The memory 125 may comprise a representation of stimulus commands (or data) to control the stimulus source 120. These stimulus commands may be associated with, or integrated in, the pulse sequence commands, such that, upon execution, a well-defined relationship in timing between the operations performed by the gradient coils and RF transceiver (and/or further components of the MRI system) and the stimuli provided to the patient via the stimulus source is established. For example, the stimulus commands may control or induce a change between an active state, in which a specific stimulus is performed, and a control state, e.g. in which an alternative or no stimulus for reference/control is applied, between different acquisitions of the MRI data. Metadata may be associated with the acquired MRI data to indicate a state of the stimulus source(s), e.g. parameters controlling the stimulus source and/or indications of the (type of) stimulus that was presented to the subject, at the time corresponding to the time of acquisition of the MRI data.

The computer system 122 may also be adapted to reconstruct a tomographic image from MRI data acquired from the RF transceiver. Methods for controlling the MRI system using a suitable pulse sequence and for reconstructing tomographic images from the signals collected by the RF transceiver in response are well-known in the art, and will not be discussed in detail in the present disclosure. However, it is noted that the pulse sequence will typically comprise commands or data to control the MRI system such as to collect image data (as reconstructed by corresponding processing of the acquired signals) that is sensitive to changes in the human brain due to brain activity. These changes may rely on a hemodynamic response of the brain in response to brain activity, e.g. an increased blood flow and/or changes in the rate of consumption and/or supply of resources in the blood in active regions of the brain, and/or on a blood-oxygenation level dependent signal contrast.

The computer system 122 may comprise, e.g. may implement via suitable software and/or hardware, an analysis module to analyze (a timeseries of) fMRI images, e.g. taking a schedule of stimuli provided during the acquisition of the fMRI images into account, and to output a result thereof, e.g. as explained in detail hereinbelow. The output may be provided via a user interface, stored on a fixed or removable storage medium, exported via a data communication network interface, and/or in another suitable form to make the result available to a human or machine user, e.g. for review by a clinician, storage and/or further use by a (e.g. automated) processing system.

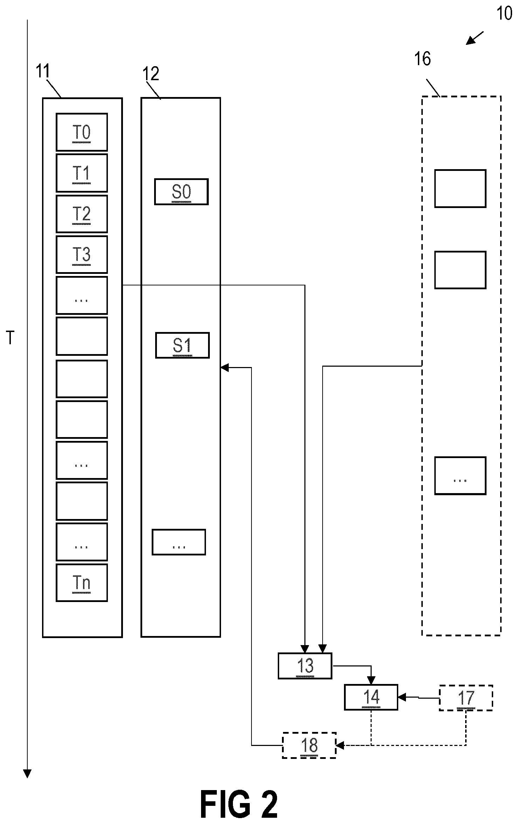

Referring to Fig 2, an illustrative method 10 for analyzing the neural response(s) of a patient to a plurality of different candidate treatment stimuli in accordance with embodiments of the present invention is shown. It is an advantage of embodiments that a variety of different stimuli may be analyzed for efficacy in treating a disorder of consciousness, i.e. as presenting in a specific patient. Even though the symptoms of a disorder of a DOC are clearly identifiable, the underlying cause(s) can vary strongly, which also affects the applicability and potential effect of any therapy. The combination of the DOC condition and its causes being difficult to quantify and/or qualify, e.g. due to a loss of (or reduction in) awareness of the patient, and the realization that typically any suitable therapy might strongly depend on the specific causes and nature of the condition of a patient, e.g. may be dependent on patient-specific disruptive injuries to the brain, results in a substantial potential for improvement by personalizing the treatment.

For example, while a noninvasive stimulation of the brain by ultrasound or TMS may benefit some patients, other patient may be better treated by a stimulation therapy program that involves visual and/or auditory stimuli. Even if such treatments were to be equally effective, the latter may still be preferable in view of the ease of application and lower requirements (e.g. of specialized or expensive equipment and/or trained personnel).

The method 10 comprises acquiring 11 a timeseries of functional magnetic resonance images of the brain of the subject using a magnetic resonance imaging system 100, e.g. an MRI system 100 as discussed hereinabove. The vertical axis T in Fig 2 illustrates a flow of time, e.g. in the acquisition of the sequence (timeseries) of images. Acquiring the timeseries of images may comprise controlling the MRI system in accordance with a pulse sequence, and collecting signals by the RF transceiver. The timeseries thus comprises a sequence of images acquired during subsequent time blocks T0, T1, ..., TN (or substantially acquired at a sequence of instants in time T0,T1, ..., TN).

Acquiring the timeseries may also comprise processing the collected signals to reconstruct an image, e.g. a tomographic image. The reconstructed image may be a volumetric (3D) tomographic image, one or more planar (cross-sectional; 2D) images, or a combination thereof. The reconstruction may, for example, be performed after acquisition of the timeseries, or may be performed during the acquisition, e.g. updating results while the examination is still in progress based on the data acquired up to that point in time. The acquired signals may be representative of substantially a same instant in time for different locations in the brain (e.g. using suitable 3D imaging sequences and/or accelerated imaging techniques), or may be representative of slightly different instants in time for different locations in the brain (e.g. using a conventional echo planar imaging technique), which may be corrected as known in the art (e.g. using a slice timing correction method). Therefore, the timeseries comprises images (2D or preferably 3D) that are substantially (or at least approximatively) representative for the imaged brain at corresponding points in time. Suitable MRI and fMRI techniques are well-known in the art, and therefore not discussed in detail, e.g. suitable pulse sequences, image reconstruction methods and/or fMRI analysis techniques (e.g. to create images representative of the brain activity, e.g. statistical maps of assumed brain activity) as known in the art can be applied. Typically, the BOLD effect is used in such techniques as proxy for neural activity in order to localize activity in the brain, e.g. by estimation from MRI images that have a suitable sensitivity to blood oxygenation dependent effects, e.g. T2^{∗}-weighted images. As an example, the acquired fMRI data of the timeseries may comprise a plurality of MRI images at a plurality of different echo times following an application of an excitation RF pulse, e.g. such that each image corresponds to a time sample point in the timeseries.

The method 10 comprises providing 12 a plurality of different candidate treatment stimuli S0, S1, ..., at different points in time and/or in different combinations, via one or more stimulus sources 120 to the patient during the acquisition of said fMRI timeseries to induce activity in the patient's brain, wherein said treatment stimuli differ in the stimulus source that is used, i.e. in the means of delivery, in the type of stimulus and/or in a parameter controlling the corresponding stimulus source(s) and/or in one or more parameters describing a property of the content delivered by or used in the stimulus.

The candidate treatment stimuli specifically comprise sensory stimuli, e.g. at least two different sensory stimuli or at least two different sets of sensory stimuli, in which the difference is defined by one or more of the factors discussed hereinabove (modality, type, content, parametrization, ...). The sensory stimuli may comprise displaying a visual cue, emitting an auditory cue, providing a touch sensation and/or providing another type of perceptual stimulus. Even though the stimuli may also comprise the stimulation of brain tissue by direct or indirect manipulation, e.g. by TMS, DBS and/or HIFU, preferably, the stimuli consist of, or comprise at least one sensory stimulus, e.g. at least two different sensory stimuli. Thus, in embodiments, stimulation of the brain by presenting sensory cues, such as video, audio, pictures and/or tactile/haptic signals, and/or olfactory or other sensory signals, can be tested for efficacy in improving the DOC condition of the patient. Embodiments may also combine sensory and (in)direct brain stimulation (i.e. by focused electromagnetic and/or mechanical signals/waves). Thus, an additive (or additional) effect of the sensory stimuli (e.g. of different types of sensory stimuli) when combined with such direct and/or indirect manipulation, or the effect of the sensory stimuli (e.g. with respect to each other) without further aid by a (in)direct brain stimulation modality can be analyzed.

For example, the sensory stimuli that are used (e.g. in a single session) may cover a spectrum of different sensory stimuli, e.g. may comprise different kinds of stimulus, such as sounds, images, videos, tactile/haptic stimulation and/or other sensory stimuli. The content of sensory stimuli may also be varied (within the same session), e.g. being of a different nature, for example having different emotional content scores (e.g. using standardized psychological grading scales, e.g. using a standardized and quantified image set such as the International Affective Picture System), different specificity to the patient, having different scores with respect to memory inducement for the patient, designed to trigger different specific emotional states, designed to evoke different thought processes or levels of thought involvement, etc..

Examples of different types of content may include, within a specific stimuli modality or combination of modalities (e.g. for the example of stimuli corresponding to the audio modality), nature or relaxing sounds, music (e.g. known to be appreciated by the patient), voices of relatives and/or friends of the patient, sounds designed to trigger a specific memory or memories of the patient, and/or other such variations. For example, the stimuli (used within the same session) may cover different sensory stimulation modalities (i.e. involve different senses) as well as cover different content, e.g. such that (e.g. the more or most) effective stimuli (or combinations thereof) can be inferred therefrom to determine (assist in determining) a treatment. A stimulus, or combination of simultaneously tested stimuli, may cover different sensory modalities (e.g. video accompanied by audio, haptic feedback with sound, ...) and/or different types of content. It will be understood that the analysis of the acquired data can enable the effect of different sensory modalities and/or types of content to be separated and/or synergic effects (e.g. where a combination is more effective than expected from the isolated effect of each component as such) to be determined, as known in the art (e.g. by applying a suitable general(ized) linear model to explain the detected brain activity in time by different stimulus sources/types), even if the isolated stimulus of a specific modality/content type is not used explicitly in the sequence, e.g. by sufficiently covering various combinations thereof to enable a decomposition.

The stimuli, e.g. different stimuli of the same modality and/or nature (e.g. emotional content, patient-specificity, ...), may also vary, over the plurality of stimuli used in the session, dependent on at least one parameter of the stimulations, e.g. an intensity, a frequency, a duration, and/or other such parameters that can vary for different stimuli of the same class. The parameter(s) may be directly related to the stimulus source, e.g. an intensity (frequency, repetition rate, focal distance, applicator position, ...) setting of a TMS apparatus (where direct stimulation types are evaluated by the method as well as sensory stimulation types), a light intensity and/or color for a light source, or a tonal frequency, intensity, dynamic changes or noise spectrum for a (simple) sound source (to only list a few examples), and/or may refer to a measure(s) relating to the content as such, e.g. by ranking sounds, voice samples, videos and/or images by one or more psychological reference scales (e.g. for valence, arousal, dominance, pleasure, concreteness, imageability, familiarity, age of acquisition, semantic size, gender association, ...) and/or other relevant content scales/parametrizations. For example, a stimulus may be assigned to a category and/or assigned a score to represent the strength of a potentially evoked emotional response, to express a measure of familiarity to the patient, and/or to codify/quantify other such qualitative or not directly (merely technically) measurable parameters.

The different candidate treatment stimuli may also be presented in different orders and/or in different combinations, e.g. to test the cumulative or temporal effect of potential combination strategies (and/or merely to achieve sufficient statistical resolving power to detect effects in the brain).

The plurality of stimuli, i.e. each stimulus or subset of stimuli (e.g. in case different combinations are tested), is provided during the acquisition 11. In other words, at least one image of the timeseries may be acquired while a direct effect of the stimulus in the brain (directly or due to an intended response of the subject's brain to the stimulus, e.g. a sensory stimulus) is presumed to be present, e.g. at least if the corresponding type of stimulation treatment and/or selection of treatment parameters is found to be effective. In addition to the at least one image acquired shortly after or during the stimulus, at least one other image of the timeseries is acquired while the effect of the stimulus can be assumed to be absent, e.g. preceding the stimulus or a sufficiently long time after the stimulus. As generally known in the art, the effect of the stimulus can be evaluated by comparing these images. It is to be noted that, typically, a relatively large number of images may be processed to determine the activity of interest in the brain accurately, e.g. comprising at least ten images in the absence of the effect and at least ten images in the presence of the effect (without implying any limitation by this example). It is also to be noted that the sensory stimulus is typically repeated, such as to repeatedly induce the brain activity of interest, to increase sensitivity by joint analysis. In other words, multiple stimuli corresponding to the same candidate treatment or stimuli class/setting under evaluation may be provided, e.g. to obtain sufficient detection power in assessing this stimulation class/setting, while, across different candidate treatment stimuli (sets), also at least one type, combination, content class, parameter, ... of the stimuli is varied to study the effect(s) of these different candidate treatment choices, e.g. to allow different approaches to be compared relative to each other.

Providing the stimulus may comprise displaying a visual cue, e.g. an instruction or instructive pictogram in an attempt to evoke a conscious decision, thought, emotion or the like upon the patient being aware and able to understand its meaning, an image intended to evoke a specific brain response (e.g. a thought, a memory or emotion), or even a cue as simple as a change in lighting conditions (e.g. a flash). Providing the stimulus may comprise emitting an auditory cue, e.g. a sound, a tone, a auditory verbal instruction, and/or a verbal or non-verbal sound intended to evoke a specific brain response. For example, such visual, auditory, audiovisual, ... stimuli may be tailored to the patient, e.g. may include pictures, voice recordings, movie clips, ... to which the patient may be assumed to respond emotionally when aware, e.g. of family members and/or other close ones. Providing the stimulus may also comprise a touch, or another cue that can be sensed by the subject's brain in any other conceivable manner, e.g. release of a molecule that can be detected by the olfactory sense.

The stimulus (or stimuli of a same type, nature, content and/or corresponding to a same parameter selection) may be repeated during a first block of time, while it may be absent during a second (e.g. next) block of time, in which these types of block may alternate for a number of times, e.g. to increase robustness of the test performed. Different types of stimulus, e.g. differing by type of content, modality, sense (sensory modality) and/or parameters of the delivery, are studied. In a blocked design, different types of stimuli may be delivered in different corresponding blocks, e.g. each block may be attributed to a specific stimulus type or parameter selection (and/or a specific simultaneous combination thereof to test this combination). The simple 'alternating' example above can readily be extended by creating a sequence of different (types of) blocks (potentially including one or more control blocks in which no stimulation or a control stimulus is applied). The order of the types of blocks can be randomized, e.g. in a balanced design to avoid cross-over effects.

It is an advantage of a blocked-trial design with alternating (or, at least, changing 'condition' from one block to the next) periods may provide a good sensitivity, e.g. a detectable signal in an image voxel or region of multiple voxels (or a detectable signal change when comparing between at least two of those periods) from which a response in the brain to the therapy stimulus can be determined. The blocks may typically correspond to different conditions or different combinations of conditions, in which the conditions being studied include the candidate treatment conditions (stimulus modality, content type, parameter selection, ...) and may typically also comprise a control condition, e.g. in which such stimuli are absent or in which similar stimuli without expected therapeutic effect are provided. By using a prolonged therapy 'block', e.g. a substantial period of time over which the stimulus is sustained and/or a plurality of the same or (qualitatively) similar stimuli are provided, a good signal quality (e.g. MRI contrast, signal to noise, ...) can be achieved to enable the detection of a response in the brain to the therapy (or an equally informative lack thereof). Furthermore, a blocked-trial design may advantageously reduce the dependence of results determined from the fMRI data, e.g. by statistical analysis, on timing-specific (or strongly timing-sensitive) parameters that may be a-priori unknown or only approximately known, such as a hemodynamic response function (e.g. latency thereof), the fluency or ease of neural signal transmission along neural pathways (which may be reduced relative to a normal state in view of the patient's condition) and/or the activation flow coupling response.

Alternatively to, or in combination with (in a hybrid paradigm), a blocked design paradigm, i.e. an "event-related" fMRI approach, may be applied. In such approach, the candidate therapeutical stimuli are considered as separate discrete events (points in time), such that neural processes of interest, e.g. cognitive processes, and the brain response associated with each stimulus event can be analyzed independently and/or by a model that does not necessarily rely on a straightforward averaging of the effect within a series of consecutive stimuli (i.e. in a single block) or a conventional mathematical extension of such averaging (e.g. fitting a block function or convolution of a block function with a response primitive, e.g. HRF). This may advantageously allow a more accurate (analytical) separation of, for example, a sensory effect of the stimulus (e.g. initial effects which generally do not involve consciousness or awareness as such) and a (e.g. cascade of) higher-level brain responses, such as triggered memories, emotional responses, thought processes, language processing and/or other such processes suggestive of consciousness. Even though explicit control events may be included, e.g. a similar stimulus to the therapy stimulus that is presumed to have no significant effect on consciousness, it is an advantage of an event-related that the isolated trials can be immersed in plenty of non-trial data sampling (time) points, e.g. such that sufficient data representative of the control condition is readily available. A more intricate analysis (e.g. as opposed to a simple binary analysis of control/effect, which e.g. may be applied to a block paradigm, without limitation thereto) furthermore allows to quantify an effect of the stimuli that occurs over an a priori unknown timespan, e.g. using a declining (linear, exponential, ...) decay model (or e.g. a delayed peak model after the event) of the sought effect after each stimulus of interest, e.g. such that each time point may be considered as a (non-binary) mix of the "control" and "event" state. This approach may also allow the assessment of a cumulative effect of stimuli, e.g. to determine a suitable therapeutic "dose" (e.g. number, frequency, total duration, ... for the therapeutical delivery of stimuli), while this may be more difficult to accurately determine using a blocked-trial design (but certainly not impossible, e.g. by using different (sets of) blocks corresponding to different parameter values, and optionally interpolating/extrapolating the detected effect sizes as function of the parameter).

For example, the stimuli provided may comprise a sensory stimulus or a series of sensory stimulus. Different types, classes or otherwise partitioned sets of stimuli can be tested, e.g. corresponding to the different treatment conditions under evaluation. The choice of the initial preferred stimuli and pattern program, i.e. the candidate treatment stimuli, and/or stimuli parameter(s) may be done by the clinician. The stimuli variables that may be varied (across different candidate trial conditions) include sensory modality, different combinations, stimulus type (e.g. content quantified or indexed by a suitable descriptive measure), the stimulation intensity, the stimulation speed and/or the stimulation period of time, and/or other variables as discussed hereinabove. For a multimodal sensory stimulation program, parameters of interest may also include the sequence of different stimuli investigated as a combination, the simultaneous combinations of (types/content/parameter selection of) stimuli being applied, the interval between sequences (e.g. between blocks corresponding to the same multimodal combination condition), and/or other such variables. The order of applying different stimulations (e.g. blocks, e.g. treatment conditions being evaluated) may also be a tunable parameter.

It will be understood that the choice of the candidate treatment stimuli and parameters under test may also, e.g. alternatively or additionally to a clinician's input, be based on randomness, e.g. may be sampled randomly (but not necessarily uniformly; e.g. a clinician's input may define a prior distribution to sample from). Furthermore, input for establishing the candidate treatment stimuli may also comprise information collected from relatives, family and/or friends of the patient, such as a favorite song, preferred music, voice recordings from such close ones, favorite smells, memorable pictures or videos (e.g. of life events or other imagery significant to the patient), and/or other information relating to personal taste, history and/or preferences of the patient.

The method comprises determining 13 an activity measure indicative of brain activity in response to each candidate treatment (class/type/set of stimuli) in at least one predetermined region of interest in the subject's brain imaged by the timeseries of fMRI images. This activity measure may be determined after acquiring 11 the timeseries and providing 12 the candidate treatment stimuli. The activity measure may also be determined during the acquisition 11, e.g. by progressively updating or recalculating the effect estimate(s), e.g. after at least some of the stimuli are provided 12, e.g. in such progressively updated or "real-time" approach (e.g. substantially real-time, in other words, sufficiently fast and/or frequent during the acquisition, within the technical and practical limitations).

It is an advantage that specific stimuli may have the power to momentarily enhance brain function, and therefore potentially behavioral responses (e.g. over a longer timeframe), in which this immediate effect can be detected by the approach discussed in the present disclosure, even if a behavioral response is not yet observable.

Furthermore, the method may also comprise obtaining 16 imaging and/or physiological (brain) data from non-MRI modalities, e.g. (a) Positron Emission Tomography image(s), and electroencephalogram (EEG) and/or other such side-information. This data can be taken into account in determining the activity measure, e.g. by using it as additional input, i.e. combined with the fMRI data, to determine relevant brain activity. For example, PET and/or EEG data may be acquired concomitantly with the fMRI data, such that relevant brain activity of the stimulus/stimuli may also be detectable from this additional source of information, e.g. to improve sensitivity and/or robustness. For illustrative purposes, as shown in Fig 2, the frequency and timing of obtaining 16 the other imaging and/or physiological data differs from that of the fMRI data. This is merely intended to indicate that no direct one-on-one relationship needs to apply, even though, obviously, it is preferable to have sufficient information to deterministically correlate the times of acquisition and localization in the brain (within the limitations of the different techniques) in order to perform a coordinated spatiotemporal analysis of all available data.

Determining 13 the activity measure for a stimulus (i.e. for a type, class or other set of stimuli corresponding to a candidate treatment under study) may comprise comparing at least one first image of the timeseries acquired while an effect of the stimulus in the brain is presumed to be absent and at least one second image of the timeseries acquired while a direct or indirect effect of the stimulus in the brain is presumed to be present (at least, if said candidate treatment corresponding to the stimulus is presumed to be, to some extent, effective).

Determining the activity measure may comprise determining neural activity, e.g. a relative change in activity compared to a control condition or other data in the acquired fMRI data that corresponds to a different stimulus or state of the patient, for at least one region of interest in the brain that is linked to conscious activity, memory activity, emotional activity and/or another higher-level brain function.

For example, at least one first image of the timeseries acquired while an effect of a (e.g. specific type of) candidate treatment stimulus in the brain is presumed to be absent and at least one second image of the timeseries acquired while an effect of the stimulus in the brain is presumed to be present may be compared to determine activity in the brain evoked by that specific type (e.g. of a specific modality, parameter selection, content type, and/or determined by another specific choice of treatment variable to evaluate) of candidate treatment stimulus. The first image may e.g. be acquired before the stimulus was applied, and the second image may be acquired during or after, e.g. shortly after, providing the stimulus, e.g. less than one minute after, e.g. preferably less than 30 seconds after, e.g. less than 10 seconds after. It is to be noted that, while the direct effect of a stimulus in the brain may be typically transient, an indirect effect can be maintained, and therefore detectable, over substantially longer periods in time (e.g. potentially extending over multiple minutes afterwards, e.g. without any limitation on sustaining neural activity over even much longer periods, even though practicality concerns may arise when a block of stimulus-induced activity extends over a time period that is too long).

Comparing at least the first and second image may be interpreted broadly, e.g. may involve any operation that involves image information from the first image and the second image to determine a qualitative or quantitative measure that indicates brain activity present in the second image where it was not, or to a lesser extent, present in the first image, or vice versa, e.g. a decreased brain activity in the second image relative to the first image. This may involve the calculation of an image subtraction, an image division or similar measure of contrast. In view of the subtle contrast differences observed in fMRI, e.g. due to the BOLD effect, it will be understood that typically a first set of images is compared to a second set of images, each corresponding to a substantial number of time points, to be able to detect the relevant contrast changes indicative of brain activity. It will also be understood that this comparison to detect contrast changes may involve a more complex model, e.g. by fitting a general linear model, generalized linear model or similar statistical model to the data, in which in addition to the indicator variables and/or parameters representative of the 'state' of a time point (e.g. in the simplest case being a binary value to distinguish the time points at which the stimuli are assumed to have effect if detectable from the time points representative of a control condition), a plurality of different parameters may be added to the model, e.g. representative of residual changes due to motion, time-dependent changes in the effect(s), constraints and/or other such variables. A predictive model of the expected brain activity may be built by taking time-dependent effects of the stimuli into account, e.g. assuming that at the time of stimulation, the effect is not yet detectable, and assigning to following images a weight based on a model of the signal response, e.g. the hemodynamical response function (HRF). Many variations are known in the art to specifically model this response. For example, the approach for a block-based design may resemble fitting a wide flat curve that is slightly offset (e.g. delayed) in time with respect to the timing of the 'active' block of stimulation (e.g. obtainable by convolution of the HRF with a boxcar function representing the timing of the block), while the approach for an event-based design may resemble fitting the data to a sequence of less easily identifiable values (e.g. each representing the sum of HRF convolutions with delta functions for indicating the preceding relevant stimuli at the time of image acquisition for which that value is determined).

As is generally well-known in the field, each voxel of the image may be fitted to such model to obtain an estimate of neural activity linked to the stimuli (or for each type of stimulus) for that point in the brain indicative of.

This may also involve suitable pre-processing and/or processing steps, e.g. to compensate for motion between the images, to correct timing, to register the images to a known coordinate framework, e.g. such that the region of interest can be easily found in the images (and the time evolution of the detected single for each point in the brain of interest can be easily retrieved and analyzed), image smoothing, and such. For example, image smoothing may be applied to allow statistically robust inference (for example, based on Gaussian Random Field properties), and/or slice timing correction may be applied to correct for small differences in the actual (center) time of acquiring slices (or other subsets) for a same image of the timeseries.

A general linear (or generalized linear or other type of) model may assign a strength and/or confidence to the brain activity in each voxel explained by the (e.g. separately for each) candidate treatment condition and/or for each region of interest (e.g. which may comprise multiple voxels, e.g. grouped based on a brain region map, a brain region segmentation and/or similar anatomical/topological information). Alternatively (or in combination), a machine learning algorithm may be trained to recognize relevant brain activity based on the input images.

For example, when side-information, such as PET and/or EEG data is also acquired, this can be inserted in the design matrix of a GLM and/or can be used to increase a weight of time points and/or spatial regions in the fMRI data based on suggestive activity detected in the PET and/or EEG data at the same time/location and/or taken into account in the analysis model in another way. In another approach, the fMRI data may be analyzed independently, and results from fMRI, PET and/or EEG analysis may be combined to determine a combined activity measure.

Determining 13 the activity measure for a stimulus (i.e. for a type, class or other set of stimuli corresponding to a candidate treatment under study) may comprise applying a trained machine learning model to the fMRI images (and/or derivative information therefrom, e.g. using a low-level salient, e.g. primitive, feature detection in the images as input) and receiving the activity measure as output. Thus, the analysis may be performed, e.g. using a processing unit or computer, by artificial intelligence. It is an advantage that a machine learning model can be trained to detect the brain activation/deactivation patterns in relevant brain regions and/or correlations between relevant brain regions. This may avoid difficulties and challenges in designing and finetuning a hand-crafted algorithm to obtain the same result, and artificial intelligence techniques have been shown to often outperform conventional algorithms in sensitivity, which may be a key factor in view of the typically noisy fMRI data from which statistically significant effects are often difficult to detect with a high degree of certainty.

It is also an advantage that a machine learning model can be trained on outputs that are not directly or easily determined by human observation or interpretation of the data. For example, a training set of data may be used that follows up on patients over a prolonged period of time after collecting the fMRI data (applying a stimuli treatment fMRI paradigm in the MRI scanner), such that actual clinical outcomes are known and available as training output values. Thus, actual (retrospective) prognosis data of the patients can be used to train a model to determine such prognosis from the initially acquired fMRI scans. As an example, a conventional trial-by-error strategy may be followed, in which a patient is assigned to a therapy program based on chance and/or the experience and judgment of an attending doctor, with the difference that at least one therapy session is performed in the MRI environment in accordance with a suitable fMRI paradigm format of the stimulation (e.g. taking balancing concerns and control data requirements into account). The patient may then be followed up by standard checkups, and the progression of the patient may be quantified based on observable (e.g. behavioral) changes over a longer timeframe. Once this data becomes available, the fMRI data and clinical outcome can be used as input and output (one training sample, obviously repeated for a number of patients) for training the machine learning algorithm. Even though the patient may be subjected to a number of different candidate treatments after a while, the machine learning algorithm may be able to detect interesting brain patterns in the fMRI scans, correlate this automatically with improved outcomes later and, given sufficient data, filter out ineffective and/or suboptimal therapies (e.g. by the training algorithm reducing the weight of any brain pattern that does not lead to promising improvements in the patients condition), at least when given sufficient training samples.

For the sake of clarity, while in this case the activity measure may be a more direct measure of clinical outcome and might not be directly interpretable (by humans) as an activity measure indicative of brain activity in (a) region(s) of the brain, it will be understood that this is, in principle, still such measure indicative of brain activity, since the machine learning algorithm is only able to determine data indicative of brain activity based on the input it receives; the activity measure is therefore, for the purposes of the present disclosure, still considered to constitute such measure of brain activity.

It is also an advantage of machine-learning approaches that side-information, such as PET and/or EEG data, can be easily inserted into the model as additional input(s) (raw and/or after suitable processing).

It will be understood that an AI-based approach can have many advantages, as discussed hereinabove. However, a more conventional approach (e.g. based on statistical maps, linear models and the like), such as discussed hereinabove, may have the advantage of better explainability, e.g. can be readily understood, evaluated and interpreted by humans (at least, for the user willing to invest an effort in the technical background). Even though significant improvements have been made in the field to create machine learning models that offer better insights in the implicit decision making process applied by the trained algorithm, e.g. to reduce the black-box effect of machine learning, a human-designed algorithm may often still be considered as being better documented and interpretable, which may have implications for quality assurance, accountability and due diligence.

The candidate treatment stimuli may be provided 12 at different times during the acquisition 11 of the timeseries, and/or in different combinations at different times during the acquisition; in which a model may be used to analyze the produced fMRI images. The model may use a conventional, e.g. GLM, approach or may be a trained machine learning model. For example, the candidate treatment stimuli (and/or blocks of stimuli) may be codified in a design matrix of a GLM model, e.g. to provide a suitable representation of the mixing and/or timing of the treatment conditions as they apply to each acquisition (time sample). The activity measure may be determined 13 by detecting a change in the acquired images of the timeseries in the at least predetermined region as dependent on the different times at which the different stimuli and/or different combinations of stimuli are provided.

By determining the activity measure (e.g. correlated with the candidate treatment stimuli) to indicate the neuronal activity in one or more regions of interest in the subject's brain, the effectiveness of the (i.e. each type of) stimulus on the disordered and/or injured brain of the patient can be determined, where an effect might typically not be readily obvious by external responses or observation of the patient.

Determining 13 the activity measure indicative of brain activity in predetermined regions of interest may also comprise, additionally or alternatively, an attentional network (e.g. resting-state or default-mode network) activation analysis, e.g. in which estimated activity (e.g. BOLD values) of different regions of interest are considered in relation to each other. Particularly, determining 13 the activity measure may comprise performing an attentional network activation analysis. Thus, the measure of brain activity (or a component thereof) may also indicate a relation between the activity in different regions, instead of a mere indication of activity in a single corresponding region (or a list of activities in different regions). Thus, the activity measure for the at least one predetermined region may also consist of or comprise a measure of causal or correlated activation (and/or deactivation with respect to a reference control state) of a plurality of different regions, e.g. to quantify a response of different brain regions working together in response to the stimulation. For example, network analysis and/or resting-state functional magnetic resonance imaging (rs-fMRI) techniques (e.g. for evaluation of changes in activity on a generally longer time scale, e.g. in addition to a more conventional fMRI analysis to detect changes in shorted time scales) may be applied to map functional connections, e.g. inferred from correlations between the time series for different regions.

The predetermined region in the brain may comprise brain regions typically involved in consciousness, emotional response, memory, awareness and/or similar higher level functions of the brain (e.g. as opposed to limited low-level processing of sensory information and other such automatic functions). The predetermined region in the brain may comprise the brainstem and/or part(s) thereof, e.g. the reticular activating system (RAS) structure, which is known to be involved in maintaining consciousness (e.g. activation of this structure and/or connectivity, e.g. correlated activity of this region with another region or regions of interest, e.g. in the cortex, may be considered to be indicative of consciousness).

Such regions of interest comprise, for example, the frontal cortices (and/or part thereof), the anterior cingulate cortex (ACC; and/or parts thereof), the parietal cortices (and/or part thereof), the temporal cortices (and/or part thereof), the thalamus (and/or part thereof) and/or the caudate nucleus (and/or part thereof). For example, a (e.g. transiently; e.g. statistically significant) increased and/or decreased BOLD signal response to a stimulus under study in any, all or subset thereof may be indicative of consciousness and/or alertness (in some extent). For example, positive activation in the dorsolateral prefrontal (DLPFC), medial temporal (MTL) and lateral parietal regions may indicate some level of awareness or consciousness, while deactivation (i.e. a negative effect vs. control) may typically occur in the midline frontal and parietal regions. An increased activation in the medial temporal lobe, hippocampus, parahippocampus and/or fusiform gyri may, as a further example, be indicative of (increased) awareness. Likewise, decreasing activation in the posteromedial cortices (PMC), e.g. in the posterior cingulate gyrus and/or precuneus, may also be indicative of improved awareness (e.g. of top-down regulation). Instead (or in addition to) analyzing a response in such regions, a network analysis may also be performed, e.g. to quantify a coordinated response (activation and/or deactivation, and/or combinations thereof) of such (illustrative) brain regions.

For example, the brain regions of interest (individually or as coordinated network) may comprise regions involved in the default mode network (DMN), or medial frontoparietal network. This network may specifically comprise the previously mentioned medial prefrontal cortex, the posterior cingulate cortex, the precuneus and the angular gyrus. Deactivation of the DMN may generally be indicative of an increase in attentional focus and/or (active) awareness, e.g. may indicate deliberate thoughts. A significant activation of the DMN is not necessarily contra-indicative. For example, while the DMN may indicate a focus (if any) that is more directed inward (e.g. day dreaming), it may also be indicative of remembering the past, thinking about close ones, conceptual cognitive activity and/or other such more introspective forms of consciousness. In other words, the measure for brain activity is not necessarily limited to a measure for activation and/or deactivation of specific brain regions, but may also express a significant change with respect to baseline as indicative of a potentially therapeutic treatment stimulus, e.g. in view of an implicated function in the neurological basis for the "self', i.e. Theory of Mind, an involvement in reflection on emotions of oneself an/or others, in (thought and/or feeling of) social isolation (which may be particularly relevant here), as well as various (episodic) memory functions, e.g. which may be (intentionally) triggered by at least some of the candidate stimulation strategies being explored.

Other networks of interest may include the frontoparietal network, and/or the dorsal and/or ventral attention networks (resp. FPN, DAN, VAN).

The network activation analysis may comprise the calculation of one or more measures of connectivity and/or coherence that express the joint (coherent) activity of different predetermined regions in the brain. For example, functional connectivity measures may be determined (without limitation thereto) on the basis of a graph analysis, in which nodes of the graph represent different brain regions and the edges of the graph represent the connections between these regions). Simpler (exemplary) approaches may be based on measures of correlation or association between timeseries representative of different brain regions, e.g. may be based on a covariance matrix calculated for a matrix X = (X₁,X₂,...,Xₙ)^{T}, where each random variable Xᵢ represents a timeseries of detected activity in a corresponding brain region, e.g. by averaging the acquired fMRI signals over the voxels associated with the brain region.

Since a DOC patient may have suffered severe brain injuries or may have a dislocated, disordered, warped and/or otherwise abnormal brain topology (e.g. potentially missing functional parts and/or showing locally voids in functional physiology), advantageously, suitable brain areas of interest be determined differently from a more conventional fMRI approach. While embodiments relying on a standard anatomical atlas to identify the region(s) of interest are not necessarily excluded, it will be understood that embodiments may advantageously use patient-specific brain area mapping information.

For example, the method may comprise obtaining diffusion-weighted imaging based tractography data (dMRI) to map structural connections in the patient's brain. Such data may be acquired in the same session, or in a previous session (e.g. in view of possibly computationally demanding processing to map structural connections). Thus, an individualized brain connectome may be constructed to identify target regions in the patient's brain to analyze by fMRI to detect consciousness and/or related higher-level functions. For example, a reference (e.g. population-average) normative connectome (e.g. a standardized "wiring diagram" of the brain) may be transformed by the patient-specific dMRI data to remap target areas in the brain to potentially new locations (e.g. as the result of injuries and/or recovery therefrom). Since a reference normative connectome can be obtained at high resolution from public sources, it may be substantially easier to transform (e.g. deform) the reference connectome to fit the acquired patient data, than to produce a patient-specific connectome map from scratch.

The level of detail of the connectome (patient-specific and/or normative) may be adapted for the specific application, e.g. brain areas may need only be defined up to a level that is useful to identify the areas of interest in the patient's brain. In other words, higher resolution connectome data (which may be more difficult to accurately obtain for a single patient) is possibly not required for this approach.

Likewise, (alternatively or additionally) anatomical imaging of the brain may be used to map (the relevant) brain regions in a standard atlas onto a patient-specific image (e.g. a T1 weighted image) based on appearance, e.g. morphology. It is an advantage that such patient-specific anatomic data can be more easily obtained than tractography data, might already be acquired for use in processing of the fMRI data by default (e.g. for normalization), and can be processed relatively quickly. Even though the appearance of the brain as such does not necessarily reveal shifts or remapping of the functional connections (detectable by tractography), deformation and/or other changes in morphology (relative to the 'normal' brain) as the result of trauma can be easily detected and accounted for in this manner. Tractography algorithms, in the other hand, allow the detection of white-matter connections (within the technical boundaries of resolution and accuracy of detection) between different regions to be identified. It will be understood that both approaches can also be advantageously used in combination, e.g. determining a structural mapping from anatomical images and a connection diagram between regions identified that way based on tractography.

The method comprises outputting 14 at least one value, based on said activity measures, that is indicative of the neural response for each (e.g. individually) of said candidate treatments, of a ranking of said candidate treatments, a selection of said candidate treatments, and/or one or more proposed treatment plans.

The method may also comprise 17 obtaining, e.g. during and/or after the fMRI acquisition, additional information for assessment of the effect of the stimuli, e.g. face reaction data, eye movements, pupil dilation, muscle reaction and/or physiological reactions, such as breathing rate and/or heart rate. This may also (additionally or alternatively) comprise a score(s) based on a clinical behavioral scale, e.g. by human observation of the patient. These types of additional information may be taken into account in determining and outputting 14 the at least one value, e.g. such as to obtain a multimodal assessment using neuroimaging together with other available data indicative of a patient's response to the stimulation program. It will be understood that some of these examples can be obtained in real-time and/or at a sufficient frequency during the fMRI timeseries acquisition, e.g. may be directly taken into account in the fMRI analysis, while other examples may only allow for infrequent or even a single time sample, e.g. a behavioral grading after completion of the fMRI test. Nonetheless, such information that is not sufficiently resolved in time to detect the different effects of the candidate treatment stimuli may still be taken into account in the end result, i.e. the output, for example to compare the results to the results obtained in other (e.g. previous) sessions performed for the same patient.

For example, a multimodal assessment using neuroimaging (fMRI, possibly in combination with EEG and/or PET, ...) together with standardized clinical behavioral scales may be used to provide a more robust evaluation of the effect of the stimulation and thus, subsequently, to optimize a personalized therapy.

The output may also quantify or express an evaluation of prognosis and rehabilitation of the patient by evaluating the current state of the patient relative to a reference (e.g. initial) patient's state.

For example, when sessions in which the method is applied are repeated over time, previous output results (e.g. the activity measures expressing brain activity resulting from candidate treatments and/or the value or values indicative of these neural responses, candidate treatment rankings, etc.) and/or an initial value (e.g. from a first session, possibly a scam session without delivering effective stimuli, and/or obtained by a dedicated fMRI scan protocol to determine an initial reference state of the patient, e.g. by rs-fMRI) may be taken into account to determine a progress of the patient and/or to prognose a future progress. This may be done by a deterministic algorithm, e.g. using time series analysis, regression techniques or the like and/or by a machine learning technique, e.g. using the outputs of different sessions of the method for the same patient, i.e. based on fMRI data from different sessions, as input to obtain a further output, e.g. a pattern index, trained on observed (behavioral) future improvements observed in a training set of patients. It will be understood that the fMRI analysis algorithm (or further processing of the results thereof) may also be adapted to take previous results into account if available, e.g. a machine learning algorithm may be trained to receive further inputs representative of one or more previous sessions to take the patient's history into account (e.g. with this further input or inputs set to a missing-value indicator, e.g. NaN, for the first session).

In a method in accordance with embodiments of the present invention, the steps of determining 13 the activity measure(s) may be performed during the acquisition 11, e.g. and during the sequence of provided 12 stimuli, so as to progressively recalculate and/or update activity measure(s). At least some of the processing can run in parallel with the fMRI acquisition (including the delivery of the stimuli in accordance with the fMRI paradigm), e.g. for the sake of efficiency. Thus, by incremental updates, the results can be live reviewed by a clinician to determine which candidate treatments show promising results, e.g. to allow a change to another strategy, e.g. (a) different candidate treatment(s), different stimulus parameters, and/or other different parameters of the candidate treatment.

The method may also be adapted to optimize 18 the candidate treatment stimuli during the acquisition, e.g. based on the progressively updated and/or recalculated activity measure(s) (and/or output values). For example, this may comprise scheduling (a subset of) the plurality of different stimuli based on intermediate results obtained from the fMRI data already acquired. For example, the number, length, intensity and/or similar parameters of stimuli (corresponding to a predetermined candidate treatment) may be increased if the corresponding (intermediately calculated) activity measure shows no or only a weak effect, e.g. to increase the statistical power in testing for that candidate treatment. Additionally or alternatively, the test strategy may be focused (by re-evaluating the schedule of stimuli yet to be provided) on those candidate treatment stimuli that show promising results, e.g. for which the intermediately calculated activity measure indicates a relatively strong neural response in the brain region(s) of interest (and/or an indicative increase or decrease in neuronal network correlation). This may also comprise tuning one or more parameters of the stimulation to detect an optimal value or value range, e.g. using a gradient descent method (for continuously tunable parameters).

It is also an advantage of such dynamic approach that a wide(r) variety of candidate treatment stimuli or combinations thereof can be tested within time constraints for the fMRI examination. For example, a binary decision tree of possible treatment stimuli types (parameter ranges, content types, combinations of simultaneous stimuli, ...) may be explored by testing two options and pruning away the least effective (e.g. for which the output value representative for the neural response and/or the activity measure is the lowest), and continuing with providing 12 different stimuli in the remaining branch of the tree, e.g. comparing two different options for the determined most effective candidate treatment. For example, at the beginning of the procedure, visual stimuli may be compared to auditory stimuli. When visual stimuli are found to be more effective in triggering relevant brain activity, the effect of static images may be compared video clips. If static images are found to be more effective, a comparison can be made between images with a high degree of patient-specificity (e.g. family pictures) to non-specific images (e.g. pictures of random people or events, preferably rated for a same arousal, valence and/or other psychological dimensions as te patient-specific set). In a next step, the silent display of images can be compared to images accompanied by audio, and so on. It will be understood that this example of a decision tree approach can be easily extended to more than two alternatives being explored at each step, e.g. three or more different candidate treatments, and focusing on the most effective (e.g. best out of three) or the more effective (e.g. best two out of three) in the further procedure.

In a second aspect, the present invention relates to a computer program product comprising machine executable instructions for execution by a processor, wherein execution of the machine executable instructions causes the processor to perform a method in accordance with embodiments of the first aspect of the present invention.

In a third aspect, the present invention relates to a device for analyzing neural responses of a patient to a plurality of different candidate treatment stimuli for treating a disorder of consciousness. The device may be, specifically, adapted to perform a method in accordance with embodiments of the first aspect of the present invention. Details of features and/or further (e.g. optional) features of the device will be clear in view of the present disclosure hereinabove, e.g. relating to a method in accordance with embodiments, and/or vice versa.

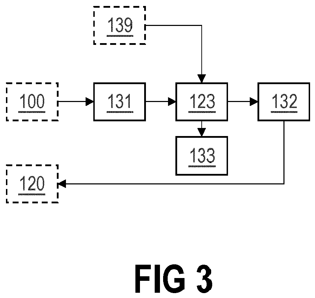

Fig 3 shows schematically an illustrative device 122, e.g. a computer system, in accordance with embodiments of the present invention.

The device 122 comprises an input 131 for receiving a timeseries of functional magnetic resonance images of the brain of the patient acquired using a magnetic resonance imaging system 100. The device 122 comprises a first output 132 for providing (and/or controlling the delivery of) a plurality of different stimuli at different points in time and/or in different combinations via one or more stimulus sources 120 to the patient during the acquisition of the timeseries to induce activity in the patient's brain, in which the plurality of different stimuli comprises sensory stimuli, i.e. signals (when delivered by the stimulus source in suitable form) that are suitable for being directly perceived by the brain via the patient's sensory organs. The different stimuli correspond to different candidate treatment conditions, e.g. an audio stimulation condition, a visual stimulation condition, an olfactory stimulation condition, an audio and/or video stimulation by familiar content (e.g. voice recordings, pictures and/or video clips of family members and/or friends), a specific form of tactile stimulation, a class of odors, and/or other such options that share (within the treatment condition) a common sensory modality, a content type, a parameter of stimulus delivery, a parameter of the content, ....

The device may comprise the stimulus source(s) 120, or may comprise a suitable hardware interface to connect to such stimulus source(s) and control the operation thereof, e.g. a video output, an audio output, and/or a bus interface, a network interface, a wireless interface, a cable connection and/or the like to control an external device for providing stimuli to the patient while in the MRI environment.

The different treatment stimuli differ in the stimulus source that is used, in the type of stimulus and/or in a parameter of the stimulus. However, for each such treatment condition, typically multiple stimuli may be provided, e.g. the same stimulus may be repeated and/or a plurality of stimuli that have the treatment condition in common may be provided. For example, the plurality of different stimuli may differ from each other, i.e. from one treatment condition to another, based on their sensory modality and/or the specific combination thereof (e.g. auditive only, visual only, audiovisual, haptic, ...). The plurality of different stimuli may also (additionally or alternatively) differ (between different treatment conditions) based on a content type of content delivered via the stimulus, such as abstract images, generic (e.g. stock) photographs, (the patient's) family photos, landscape photos, voice recordings, sounds of nature, .... The plurality of different stimuli may differ from each other (i.e. between conditions) based on a patient-specific level of memory association for the content based on memories of, or familiarity to, the patient. For example, generic images, sounds, videos, odors and/or other content (e.g. using other sensory modalities) may represent a low level of association, while photos, audio recordings, video clips and such of family members, friends, colleagues and such, of familiar places and/or objects, and/or odors (e.g. of perfume, food, ...) may represent a high level of association. The plurality of different stimuli may differ from one another based on a gradient of the content as function of arousal, valence and/or another psychological grading parameter. Examples include, for example, images graded (e.g. by a test panel) for emotional content (e.g. of neutral faces, angry faces, happy faces, ..., and/or of e.g. abstract to concrete imagery). The plurality of different stimuli may also differ in one or more technical parameters of the stimulus delivery, e.g. a frequency of stimuli (e.g. displayed images), an intensity of stimuli (e.g. a volume of sound), an inherent property of a specific type of stimuli (e.g. a frequency of an audio tone, a color of an image, ...), rTMS configuration (if TMS is also investigated, e.g. in combination with and/or compared to sensory stimuli; e.g. a stimulation locus, a field intensity, a stimulation frequency, ...) and/or other such technical parameters.

The first output 132 may comprise an interface for controlling a visual, auditory, tactile, haptic, olfactory and/or another sensory modality stimulator, and/or the stimulus source 120 may comprise a visual, auditory, tactile, haptic, olfactory and/or other sensory modality stimulator. In an embodiment, the stimulus source may comprise at least two different sensory modality stimulators, e.g. for visual, auditory, tactile, haptic, olfactory and/or other form of sensory feedback, and/or the first output may be adapted to output at least two such different (modality) sensory signals.

The stimulus source may comprise a light source, a display monitor, a projection screen and/or another visual feedback device to provide a visual signal to the patient. The stimulus source may comprise a speaker, headphones and/or another auditory feedback device to provide an auditory signal to the patient. The stimulus source may comprise a (MR-compatible) controllable odor stimulus system, e.g. an olfactometer for stimulating olfaction, e.g. by using one or more liquid odorants. The stimulus source may comprise a haptic feedback device, a controllable pressure outlet, a heater and/or cooler (e.g. to apply a heat or cold sensation, e.g. localized on a part of the skin of the patient), a vibrating element, and/or another somatosensory feedback device.

The stimulus source may also comprise a direct or indirect brain stimulator, e.g. to modulate activity of the brain by electric, magnetic and/or mechanical stimulation of brain tissue, such as a (repetitive) transcranial magnetic stimulation coil (and apparatus), an electric brain stimulator, e.g. using electrical brain stimulation, deep brain stimulation, focal brain stimulation, an electroconvulsive therapy device, and/or other means for (in)direct stimulation of brain tissue.

The device further comprises a processor 123 for determining an activity measure indicative of brain activity in response to each candidate treatment condition in at least one predetermined region of interest in the patient's brain based on the acquired timeseries.

The processor may be adapted to determine the activity measures by fitting a general linear model, generalized linear model and/or (other type of) statistical model to the acquired timeseries. Alternatively or additionally, the processor may be adapted to determine the activity measures by evaluating a trained machine learning model with the acquired timeseries and/or information derived therefrom as input and the activity measures (and/or the output value) as output.

The processor may be particularly adapted to determine the activity measure, e.g. for each candidate treatment condition, in at least one predetermined brain region associated with consciousness, awareness, memory, emotion and/or a higher brain function, and/or combinations thereof. The at least one predetermined brain region may comprise one or more of: the reticular activating system, the anterior cingulate cortex, the thalamus, the caudate nucleus, the hippocampus, the parahippocampus, the fusiform gyrus, the posterior cingulate gyrus, the precuneus, one or more regions of the frontal cortex, of the parietal cortex and/or of the temporal cortex, the posteromedial cortex, the dorsolateral prefrontal cortex, and/or the medial temporal cortex.

The processor may also be adapted to perform a neuronal network activation analysis to determine the activity measure(s). The neuronal network analysis may determine a measure indicative of a relation between activity in different predetermined brain regions (e.g. the at least one predetermined brain region discussed hereinabove, or a subset thereof). The relation between activity in brain regions may comprise activity of the medial frontoparietal network, the frontoparietal network, the dorsal attention network and/or the ventral attention network. This relation may comprise an increased correlation or other measure of cohesion (e.g. mutual information, ...) of observed brain activity in the regions, a decrease thereof, or a combination thereof (e.g. a predetermined part of the network increasing coherence and another decreasing), in response to the candidate treatment condition (e.g. as compared to a control condition).

The predetermined brain region(s), e.g. to be analyzed in isolation and/or in a network analysis approach, may be located, by the processor, based on a standard brain region atlas, and/or on a reference brain map of the patient. For example, the device may comprise an input to receive an anatomical reference magnetic resonance image (e.g. a T1 weighted image) of the patient (e.g. from the MRI system), and the processor may determine a mapping of a standard (e.g. defined for a reference population) brain region atlas to the patient-specific anatomic image (or vice versa), e.g. using coregistration and/or normalization techniques. The device may comprise an input to receive diffusion-weighted images of the patient (e.g. from the MRI system) and the processor may be adapted to perform a tractography analysis to detect the predetermined regions of interest and functional connectivity between the regions, e.g. to determine a network for said network analysis. It will be understood that atlas-based, patient-specific anatomical information and/or patient-specific tractography may also be combined.

The processor may also be adapted to control the delivery of the stimuli, e.g. via the first output 132 and the stimulus source(s) 120. The processor may also be adapted to control the fMRI data acquisition, via a suitable device interface to the MRI system, e.g. to emit trigger(s) to acquire image(s) and/or control parameters of the MRI acquisition. The processor may be adapted to coordinate the timing of the stimuli and acquisitions and/or to receive timing information (e.g. from the MRI system), so as to determine a relation between points in time of acquisition and of stimulation.

The processor 123 may also be adapted to generate a (e.g. timing and/or composition) schedule for the stimuli to be provided via the stimulus source(s), and e.g. control the execution of this schedule via the first output.

For example, the plurality of different stimuli (or sets of different stimuli corresponding to the treatment conditions under examination) may be provided at a plurality of different times during the acquisition, e.g. as controlled by the processor 123, in accordance with a functional magnetic resonance imaging block paradigm design, a functional magnetic resonance imaging event-related paradigm design and/or a hybrid paradigm combining stimulation (time) blocks and isolated stimulation events (trials).

The device may also comprise a further input 139 for receiving, during and/or after the fMRI acquisition, assessment data from the patient. This information may be received as input from a user (e.g. using a user interface, e.g. via a keyboard and/or mouse, a touchscreen, etc.) and/or obtained from a data acquisition device. For example, the further input 139 may receive clinical behavioral scoring data (a GCS coma scale score), e.g. entered via a suitable user interface by an attending clinician. The further input 139 may comprise face reaction data, muscle reaction data, eye movement data and/or pupil dilation data, e.g. received from an eye tracker device, an electromyography device and/or a camera observation system equipped with a suitable image (video) processing system (e.g. software) to infer such data from camera observation images of the patient during the fMRI examination. The further input 139 may receive physiological data, such as heart rate, an electrocardiogram, respiration data, blood pressure, and/or other such data, from a heart monitor (e.g. electrocardiography system), breathing monitor (e.g. breathing belt) and/or suitable physiological monitoring device.

The further input 139 may be adapted to receive data during the acquisition, e.g. such that changes thereof in time during the examination, e.g. indicative of the effects of the (e.g. each) treatment conditions, may be taken into account. The further input may also be adapted to receive data after the acquisition, e.g. for a parameter that is difficult to acquire with sufficient temporal resolution, e.g. a behavioral scale score. Even though this may generally be less useful to detect effects of the different treatment conditions (with respect to each other), it may still be used in processing, e.g. to adjust weights and/or parameters of the fMRI analysis and/or to compare current results to those of previous sessions (e.g. for patient follow-up and prognosis). The further input 139 may be adapted to receive further imaging and/or physiological data, e.g. using a non-magnetic-resonance diagnostic imaging modality, such as Positron Emission Tomography, electroencephalography and/or magnetoencephalography, and taking the further data into account in determining the activity measure(s) and/or output value.

The processor may be adapted to determine the activity measure during the acquisition, e.g. while further fMRI data is still to be received via the input 131, so as to progressively recalculate and/or update the activity measures. For example, the processor may be adapted to optimize the plurality of different stimuli and/or a scheduling thereof, e.g. by adjusting the timing and/or composition schedule for the stimuli to be provided via the stimulus source(s) and control the (further) delivery of stimuli in accordance therewith via the first output. Thus, the stimuli and/or schedule thereof may be optimized based on the progressively updated and/or recalculated activity measures so as to adapt the sequence of stimuli yet to be provided.

The device further comprises a second output 133 for outputting at least one value, based on the activity measures, that is indicative of the neural response for each of the candidate treatments, of a ranking of said candidate treatments, and/or of a selection of said candidate treatments. The processor may thus be adapted to generate this at least one value to be provided as output. The at least one value may comprise the activity measure (e.g. an activity measure for each treatment condition), and/or may process this activity measure(s) into a suitable form, such as a ranked (e.g. sorted) list of the candidate treatments as function of the determined activity measure, a selection of the more or most effective candidate treatments (e.g. with the highest activity measure observed), one or more proposals for treatment strategy in which one or more of the most effective candidate treatments are used, etc.

The processor may also be adapted to determine the at least one value (or part thereof) as a prognosis indicator indicative of a prognosis of the patient, e.g. by taking the output value and/or activity measures determined in a previous session for the same patient into account, e.g. to compare a response to a treatment condition to a response to this treatment condition determined in a previous use of the device for the same patient.

In a fourth aspect, the present invention relates to a magnetic resonance imaging system 100 comprising the device 122, e.g. the computer system, in accordance with embodiments of the third aspect of the present invention. For example, reference is made to the illustrative MRI system 100 discussed hereinabove.

Other features, or details of the features described hereinabove, of a device, system and/or computer-program product in accordance with embodiments of the present invention shall be clear in view of the description provided hereinabove relating to a method in accordance with embodiments of the present invention, and/or vice versa.

## Claims

1. A device (122) for analyzing neural responses of a patient to a plurality of different candidate treatment stimuli for treating a disorder of consciousness, the device comprising:
- an input (131) for receiving a timeseries of functional magnetic resonance images of the brain of the patient acquired using a magnetic resonance imaging system (100),
- a first output (132) for providing a plurality of different stimuli at different points in time and/or in different combinations via one or more stimulus sources (120) to the patient during the acquisition of the timeseries to induce activity in the patient's brain, wherein said plurality of different stimuli comprises sensory stimuli, and said different stimuli correspond to different candidate treatment conditions,
- a second output (133) for outputting at least one value that is indicative of the neural response for each of the candidate treatment conditions, of a ranking of said candidate treatment conditions, and/or of a selection of said candidate treatment conditions,
- a processor (123) for determining an activity measure indicative of brain activity in response to each candidate treatment condition in at least one predetermined region of interest in the patient's brain based on the received timeseries, and for generating and outputting said at least one value, via said second output (133), based on said activity measures,
wherein said treatment stimuli differ in the stimulus source that is used, in a type of stimulus and/or of its content, and/or in a parameter of the stimulus.

2. The device of claim 1, comprising said one or more stimulus sources (120), wherein said one or more stimulus sources (120) comprise a visual, auditory, tactile, haptic, olfactory and/or another sensory modality stimulator.

3. The device of claim 2, wherein said one or more stimulus sources (120) further comprise a direct or indirect brain stimulator to modulate activity of the brain by electric, magnetic and/or mechanical stimulation of brain tissue.

4. The device of any of the previous claims, wherein said processor (123) is adapted to determine the activity measures by fitting a general linear model, generalized linear model and/or statistical model to the received timeseries, and/or wherein processor is adapted to determine the activity measures by evaluating a trained machine learning model with the received timeseries and/or information derived therefrom as input and the activity measures and/or the output value as output, and/or wherein said processor is adapted to perform a neuronal network activation analysis to determine said activity measure.

5. The device of any of the previous claims, wherein said processor (123) is adapted to determine the activity measure in the at least one predetermined brain region associated with consciousness, awareness, memory, emotion and/or a higher brain function, and/or combinations thereof.

6. The device of any of the previous claims, wherein said processor (123) is adapted to locate said at least one predetermined brain region based on a reference brain map of the patient determined from an anatomical reference magnetic resonance image of the patient and/or by performing a tractography analysis of diffusion-weighted images of the patient.

7. The device of any of the previous claims, comprising a further input (139) for receiving, during and/or after receiving the timeseries, assessment and/or physiological data of the patient, wherein said assessment data comprises clinical behavioral scoring data, face reaction data, muscle reaction data, eye movement data and/or pupil dilation data, and/or for receiving further imaging data,
wherein said processor (123) is adapted to take said assessment, physiological and/or further imaging data into account in determining said activity measure and/or output value.

8. A magnetic resonance imaging system (100) comprising the device of any of the previous claims.

9. A method (10) for analyzing neural responses of a patient to a plurality of different candidate treatment stimuli for treating a disorder of consciousness, the method comprising:
- acquiring (11) a timeseries of functional magnetic resonance images of the brain of the patient using a magnetic resonance imaging system (100),
- providing (12) a plurality of different stimuli (S0, S1) at different points in time and/or in different combinations via one or more stimulus sources (120) to the patient during the acquisition of said timeseries to induce activity in the patient's brain, wherein said plurality of different stimuli comprises sensory stimuli and wherein said different stimuli correspond to different candidate treatment conditions,
- determining (13) an activity measure indicative of brain activity in response to each candidate treatment condition in at least one predetermined region of interest in the patient's brain based on said timeseries,
- outputting (14) at least one value, based on said activity measures, that is indicative of the neural response for each of said candidate treatment conditions, of a ranking of said candidate treatment conditions, and/or of a selection of said candidate treatment conditions,
wherein said treatment stimuli differ in the stimulus source that is used, in the type of stimulus and/or of its content, and/or in a parameter of the stimulus.

10. The method of claim 9, wherein said plurality of different stimuli are distinguished from each other based on their sensory modality and/or specific combination thereof, on a content type of content delivered via the stimulus, on a patient-specific level of memory association for the content based on memories of or familiarity to the patient, on a grading of the content as function of arousal, valence and/or another psychological grading parameter, and/or on one or more technical parameters of the stimulus delivery.

11. The method of claim 9 or 10, wherein each of said plurality of different stimuli is provided at a plurality of different times during said acquisition (11) in accordance with a functional magnetic resonance imaging block paradigm design and/or a functional magnetic resonance imaging event-related paradigm design.

12. The method of any of the claims 9 to 11, wherein said at least one predetermined brain region comprises one or more of: the reticular activating system, the anterior cingulate cortex, the thalamus, the caudate nucleus, the hippocampus, the parahippocampus, the fusiform gyrus, the posterior cingulate gyrus, the precuneus, one or more regions of the frontal cortex, the parietal cortex and/or the temporal cortex, the posteromedial cortex, the dorsolateral prefrontal cortex, and/or the medial temporal cortex, and/or wherein determining (13) said activity measure comprises performing a neuronal network activation analysis to determine a measure indicative of a relation between activity in different predetermined brain regions and/or of the medial frontoparietal network, the frontoparietal network, the dorsal attention network and/or the ventral attention network.

13. The method of any of the claims 9 to 12, wherein said output value comprises a prognosis indicator indicative of a prognosis of the patient as determined by taking the output value and/or activity measures determined in a previous session of performing the method for the same patient into account.

14. The method of any of the claims 9 to 13, wherein the steps of determining (13) the activity measure is performed during the acquisition (11) so as to progressively recalculate and/or update the activity measures, the method further comprising optimizing (18) the plurality of different stimuli and/or a scheduling thereof based on the progressively updated and/or recalculated activity measures, so as to adapt the sequence of stimuli yet to be provided (12).

15. A computer-program product adapted to perform a method in accordance with any of the claims 9 to 14.
